(19) Europäisches Patentamt
European Patent Office
Office européen des brevets

(11) EP 2 982 652 A1

(12) **EUROPEAN PATENT APPLICATION**

(43) Date of publication:
**10.02.2016 Bulletin 2016/06**

(21) Application number: **14180393.2**

(22) Date of filing: **08.08.2014**

(51) Int Cl.:
***C01G 49/06*** (2006.01)
***C01G 49/08*** (2006.01)
***C09C 1/24*** (2006.01)
***A61K 49/18*** (2006.01)
***C09C 3/10*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität für Bodenkultur Wien**
**1180 Wien (AT)**

(72) Inventors:
- **Reimhult, Erik**
  **1220 Wien (AT)**
- **Zirb, Ronald**
  **1230 Wien (AT)**

(74) Representative: **Sonn & Partner Patentanwälte**
**Riemergasse 14**
**1010 Wien (AT)**

(54) **Ultra-dense shell core-shell nanoparticles**

(57) The invention provides a method of producing inorganic core particles comprising a dispersant shell, comprising a dispersant molecule in a high surface covering density on the inorganic core, comprising the steps of providing an inorganic particle, ligating an organic linker onto an inorganic particle, thereby obtaining an inorganic core linker coated particle, providing a fluidized dispersant, preferably in form of a melt, suspension or solution, binding the fluidized dispersant to the organic linker, thereby obtaining the inorganic core particles comprising a dispersant shell, as well as particles obtainable by such a method.

Fig. 9

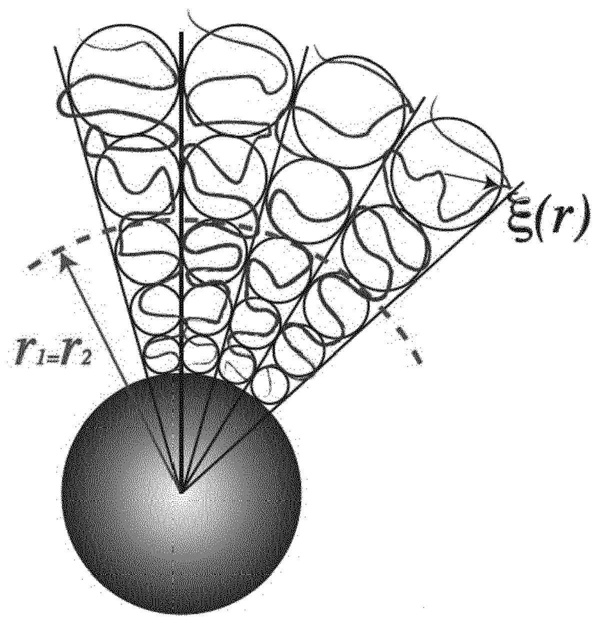


EP 2 982 652 A1

## Description

### Field of the invention

The present invention relates to the field of polymer coated nanoparticles.

### Background of the invention

Polymer shell-inorganic core nanoparticles are sought for their unique properties applicable for example in biomedical imaging, drug delivery and new therapeutics. They are also investigated as additives to improve bulk material properties. The creation of a dense functional polymer shell that shields the inorganic core from the environment is crucial to function.

Superparamagnetic nanoparticles (NPs), such as $Fe_3O_4$ NPs, with core diameters of 3-15 nm, are used in a rapidly expanding number of applications in the biomedical field; the most common include magnetic cell labeling, hyperthermia, drug delivery, and as contrast agents for magnetic resonance imaging. Iron oxide cores are coated with polymer dispersants, to enable dispersion of NPs in aqueous solutions and organic matrices. NPs will rapidly aggregate without a polymer shell; attractive interactions between NPs or between NPs and biological molecules lead to aggregates, precipitates and loss of function. With recent improvements in the synthesis of NPs, there has been a push towards more and more well-defined, core-shell particle architectures.

Monodisperse spherical iron oxide NPs can be synthesized with monodisperse magnetic and other physical properties; these NPs are stabilized with an irreversibly anchored shell of linear, end-grafted polymer dispersants of sufficient thickness to ensure that the particle properties remain monodisperse during application. This defined core-shell architecture enables the prediction of all colloidal properties and serves as a platform for further defining and designing physical, chemical and biological interactions.

An advantage of separating the synthesis of core and dispersant with subsequent grafting of the dispersant to the core is that the hydrodynamic size of NPs can be precisely controlled in contrast to NPs with dispersant shells consisting of physisorbed high molecular weight ($M_w$) dispersants. The well-defined assembly of dispersants at the NP surface enables controlled surface presentation of functionalities. NP size, stability, dispersant shell thickness and control over functionalities presented at the NP surface are the factors that critically determine NP performance in a biological fluid and also for many non-biological applications. The maximum achieved grafting densities on planar substrates of PEG(5kD) are in the range of 0.4 chains/nm$^2$ (on $TiO_2$) [J. L. Dalsin et al. Messersmith, Langmuir 2004, 21, 640-646.]; and only ~0.5 chains/nm$^2$ has been reported on iron oxide NPs after ligand replacement. The separate optimization of core and dispersant creates the problem of having to graft the dispersant into a dense, brush-like shell to the core surface; this requires conditions under which an irreversible binding reaction occurs between a reactive end group on the dispersant polymer and the iron oxide core. It also requires that the reaction occurs when the polymer has a small coil size, $R_G$. The $R_G$ determines the footprint of the polymer and thereby the maximum grafting density that can be achieved.

Current ways to functionalize nanoparticles with polymer shells can be divided into two approaches: "grafting-from" and "grafting-to". The grafting from approach requires polymerization from initiators on the nanoparticle surface, a method that is difficult to control at high nanoparticle density or large volume reaction conditions, leading to polydispersity and difficulties in controlling the functionality and density of functional groups of the core-shell NPs.

The grafting-to approach uses pre-synthesized polymer ligands, which therefore can be monodisperse and added in mixtures of different functionalities to the NPs. The grafting-to approach is however limited in the polymer grafting density that can be achieved on the NP surface due to the steric constraints of the polymer coil approaching an already partially grafted NP, as the polymer requires solubility (which expands the polymer coil size) under reactive conditions to bind to the NP surface. The size of the polymer coil limits the achievable grafting density due to the effective hindrance on the particle surface.

The problem of finding appropriate reaction conditions to achieve high dispersant density on the particle surface has been shown to be increasingly problematic for state-of-the-art synthesis protocols for monodisperse inorganic nanoparticle cores that require capping agents (strongly binding ligands) during synthesis to control size and shape (Park et al., Nat. Mater. 2004, 3, 891-895); ligand controlled core synthesis requires ligand replacement from the synthesis ligand to the dispersant ligand after completed core synthesis. Monodisperse particles have the advantage that they have identical physical and chemical properties, which otherwise vary strongly for nanoparticles and affect their reproducible application. Such particles have hitherto not been demonstrated possible to functionalize with sufficiently dense covalently grafted polymer dispersant shells for biomedical (or other) applications.

Colloidal stability was previously achieved through an irreversible bond of the polymer to bare $Fe_3O_4$ NP cores by direct (one step) grafting-to using nitrocatechol anchors and a reported dispersant density of 2.6-2.8 M/nm$^2$ [E. Amstad et al., Nano Letters 2009, 9, 4042-4048; E. Amstad et al., J. Phys. Chem. C 2011, 115, 683-691; WO2011/026572 A1].

This reported dispersant density has not been observed on truly monodisperse and spherical nanoparticles, and might therefore partly be a consequence of higher surface roughness and effective surface area, which influence the calculation of surface densities. Thus, the correct grafting density taking the entire surface of the Fe core into account is effectively below 1.0 M/$nm^2$ for these nanoparticles used in the inventors' earlier work.

Synthesis of truly monodisperse (PDI<1.05) $Fe_3O_4$ core-shell NPs makes use of surfactants as capping agents, e.g. oleic acid, to control the crystal growth and sphericity [T. Hyeon et al., Journal of the American Chemical Society 2001, 123, 12798-12801; Park et al., supra]. The as-synthesized NP core then has a dense shell of oleic acid that has to be replaced by the stabilizing dispersant. However, capping agent replacement by ligands or dispersants yielded NPs with ~5 times lower PEG grafting density (i.e. densities of only 0.5 M/$nm^2$ - see table 1 herein) and thereby colloidal stability than bare particles grafted with dispersants. The lower grafting efficiency results from that grafting-to by ligand replacement of oleic acid failed fulfilling more simultaneous and mutually contradicting constraints than grafting to a bare surface. The grafting can also be inhomogenous, leading to further compromised colloidal stability.

Therefore there remains the goal to produce nanoparticles with a dense dispersant shell. Preferably these particles would also be homogenous and monodisperse.

Summary of the invention

The present inventors succeeded in providing a new manufacturing method fulfilling these needs. The present invention is defined in the claims.

In particular, the invention provides a method of producing inorganic core particles comprising a dispersant shell, comprising a dispersant molecule in a high surface covering density on the inorganic core, comprising the steps of: providing an inorganic particle, ligating an organic linker onto an inorganic particle, thereby obtaining an inorganic core linker coated particle, providing a fluidized dispersant, preferably in form of a melt, suspension or solution, binding the fluidized dispersant to the organic linker, thereby obtaining the inorganic core particles comprising a dispersant shell.

The present invention also provides a particle or a preparation of a plurality of particles obtainable by the inventive methods.

In a further embodiment the invention provides a preparation of a plurality of particles, wherein said particles comprise an inorganic core surrounded by linkers that are chemically linked to dispersant molecules, wherein the dispersant molecules (a) are at an average density of at least 1.1 dispersant molecules per $nm^2$ of the inorganic core surface , and/or (b) form a shell of constant dispersant density and a further shell of gradually reduced dispersant density with increasing distance from the inorganic core surface, wherein preferably the inorganic core is of an average size between 2 nm to 80 nm in diameter. Definition (b) relates to forming a shell of similar density to the molten polymer state. This high density shell is identifiable by small angle x-ray scattering in a solvent. Separate therefrom there is a solvable shell, distinct from the dense inner polymer shell.

An inventive preparation may comprise a plurality of particles with such dense polymer shells (a) and/or (b), wherein the inorganic core of an average size between 2 nm to 80 nm in diameter is of homogenic size in said plurality wherein the mean standard deviation of said average size is at most 10%, preferably at most 5%, even more preferred at most 2% of said average size, such as at most 0.8 nm, preferably at most 0.5 nm, preferably said preparation being obtainable by a method of as further defined herein.

Also provided is a particle comprising an inorganic core surrounded by linkers that are chemically linked to dispersant molecules, wherein the dispersant molecules are at an average density of at least 1.1, preferably at least 3.0, dispersant molecules per $nm^2$ of the inorganic core surface, and/or (b) form a shell of constant dispersant density and a further shell of gradually reduced dispersant density with increasing distance from the inorganic core surface, preferably said particle being obtainable by a method of as further defined herein. Similar as said above, this shell structure may also be due to the dispersant forming a shell of similar density to the molten polymer state identifiable by small angle x-ray scattering in a solvent and a solvable shell, distinct from the dense inner polymer shell, These particles can be provided in a preparation comprising a plurality of such particles.

Surface densities on dispersant molecules per $nm^2$ of the inorganic core surface as used herein refer to the surface of the inorganic core, and not on a spherical approximation thereof as e.g. used in reference Amstad et al. 2009, Amstad et al 2011, WO2011/026572 A1, all supra. In a direct comparison using the same analysis methods, the inventive particles have at least about 2 times higher surface densities than the particles reported previously (see comparative examples herein).

The following detailed disclosure reads on all aspects and embodiments of the present invention, irrespective of relating to a method, preparation or particle. E.g. described method steps also disclose that the resulting product can result in an element of the particle or preparation, such as specific reagents used in the method may lead to a chemical group or moiety bound to the particle or preparation. Elements described for the preparation can read on steps in the inventive manufacturing method. Furthermore described elements of the particles as such or of particles of the preparation can be elements of both groups. As such, the linker will be bound to the dispersant and in the final particle of the invention

the former linker forms an anchor group of the dispersant that binds the dispersant to the inorganic core. Therefore, all descriptions of the linker also read on such an anchor group of the dispersant in the inventive particle. Also, the invention relates to a preparation and all descriptions of particles also read on particles of said preparation.

The present invention is suitable to provide high density dispersant inorganic core nanoparticles. Furthermore it is possible to generate these particles with high size homogeneity (monodisperse) without the requirement of particle sorting by size such as by size exclusion chromatography. The generation of monodisperse inorganic particles without the necessity for size sorting is based on the metal-surfactant complex developed by Park et al. (Nat. Mater. 2004, 3, 891-895, incorporated herein in its entirety), which provides inorganic particles, that form the core of the inventive inorganic particles. However, the inventive method to obtain high density dispersant particles is not limited to metal cores - although preferred for all embodiments - and includes any inorganic material, such as semiconductors, Si and silica materials for the core particle.

**Detailed description of the invention**

The present invention for the first time manages very high surface densities. In certain embodiments, this could also be combined with the technology for producing monodisperse inorganic nanoparticles with high surface density modification by dispersants. Previously the strong bond to the capping agent reduced future ligand binding reactions.

In preferred embodiments, an inorganic particle comprising a metal in complex with a surfactant on the particle surface is provided. The surfactant is a capping agent. A capping agent is a strongly adsorbed monolayer of usually organic molecules used to aid stabilization of nanoparticles. Example surfactants are fatty acids, such as oleic acid. The surfactant may comprise a hydrophilic group and an aliphatic chain of e.g. 5 to 30 C atoms in length. The aliphatic chain may be saturated or unsaturated, comprising one or more double or triple bonds. The hydrophilic group may e.g. be a negatively charged group, or a carbonyl group, such as a carboxylate, sulphate, phosphate.

In a preferment for all embodiments and aspects of the invention, the inorganic particle core comprises preferably a metal, e.g. selected from Fe, Cu, Au, Ag, Cr, Mn, Ti, Ni, Co, or any other element of the fourth row of the periodic table, or alloys thereof. In further embodiments the inorganic particle core comprises a metalloid, a semiconductor or consists of a non-metal material. Examples are Al, Si, Ge, or silica compounds. The inorganic nanoparticle core can be a nanocrystal or a multidomain crystallised nanoparticle composed of more than one nanocrystal. Preferably the core comprises an oxide any thereof, preferably a Fe oxide, such as $Fe_2O_3$ and/or $Fe_3O_4$. In a further embodiment, the inorganic nanoparticle core comprises a hydride nitride or an iron sulfide, preferably mixed oxide/hydroxide, nitride or sulfide of Fe (II) and/or Fe (III), e.g. in the form of a nanocrystal. Preferably, the inorganic nanoparticle core is $Fe_3O_4$ (magnetite) or comprises $Fe_3O_4$ spiked with any other metal, preferably those mentioned above. "Metal" as used herein refers to the element, not to the state. The metal may be metallic (with neutral charge) or, as in most case of the present invention, non-metallic, especially in case of crystallized cationic metals.

In further preferments of all inventive aspects and embodiments, the inorganic core is magnetic, especially paramagnetic, preferably superparamagnetic. This property can be achieved by using metal nanoparticles of a material as described above, especially selected from the group consisting of iron, cobalt or nickel, alloys thereof, preferably oxides or mixed oxides/hydroxides, nitrides, carbides or sulfides thereof. In a preferred embodiment the stabilized magnetic nanoparticles are superparamagnetic iron oxide nanoparticles (SPIONs). Magnetic particles allow controlled mobility, such as for separation of enrichment of particles in a non-accessible compartment, e.g. in a patient's body by applying a magnetic field, or the capability to heat the particles by applying an oscillating field, in particular by radio wave irradiation, e.g. in the range of 10 kHz to 1000 kHz, e.g. 400 kHz.

In some embodiments of the invention, the inorganic particle core can be produced together with a surfactant. Preferably the provided inorganic particle is in complex with a surfactant on the particle surface, especially preferred in case of metal particles, and wherein ligating the organic linker onto the inorganic particle is by replacing the surfactant by said organic linker, thereby obtaining an inorganic core linker coated particle. Inorganic particles in complex with a surfactant can be obtained by contacting the particle with an inorganic particle core with a surfactant. An alternative may comprise the synthesis of the particle core in the presence of a surfactant. Such a method may comprise thermal decomposition of dissolved metal-surfactant complexes. Metal-surfactant complexes in turn - without necessary limitation to this option - may be generated by contacting a metal salt, such as a salt with a monovalent anion, preferably a halide such as a chloride (which is readily available), in particular iron chloride, with a negatively charged surfactant such as a fatty acid as mentioned above (which are also well available), thereby forming a metal-surfactant complex. The metal surfactant composition can be thermally decomposed to form the inorganic nanoparticle core. Thermal decomposition may comprise heating in a solvent to temperatures above 200 °C, in particular temperatures above 280°C, e.g. between 280°C and 350°C, preferably about 310-320°C. Suitable solvents are e.g. aliphatic hydrocarbons, such as 1-octadecene. Preferably the surfactant comprises a carboxylic acid group, which decomposes during thermal decomposition to form a metal oxide (Park et al., supra; and Hyeon et al., supra; both incorporated herein by reference). Another preferred method comprises - as shown in example 3 herein - comprises providing a metal complex, such as a metal carbonyl

(e.g. $Fe(CO)_5$, $Cr(CO)_6$ or $Ni(CO)_4$), and contacting the metal complex with a surfactant in a solvent at elevated temperatures, e.g. between 150°C and 290°C, whereby the metal particle core forms in contact with the surfactant. In these methods, particle core size can be influenced by the concentration ratio of the metal complex and the surfactant. Preferably the temperature in this method is gradually increased, e.g. by a temperature ramp, with the reaction already expected to start at a lower temperature, e.g. 170°C or lower, and for completion of the reaction is increased to a higher temperature, e.g. at least 240°C.

The inorganic core preferably has an average diameter size of 1 nm to 400 nm, preferably 1.5 nm to 100 nm, especially preferred 1.8 nm to 12 nm, 2 nm to 20 nm, 3 nm to 40 nm or 4 nm to 80 nm. Such particles can be produced by the above mentioned method. Size may further improve the magnetic properties. E.g. a sufficiently small size may be selected to achieve superparamagnetic properties. The size for such an effect is dependent on the material and can be selected by a skilled person with average skill in view of prior knowledge.

The inventive method contains the steps of

- ligating an organic linker onto an inorganic particle; if a surfactant is present according some embodiments, this will replace the surfactant by said organic linker, thereby obtaining an inorganic core linker coated particle;
- providing a fluidized dispersant, preferably in form of a melt, suspension or solution;
- binding the fluidized dispersant to the organic linker, thereby obtaining the inorganic core particles comprising a dispersant shell.

Optimal reaction conditions aim at conditions to: (1) dissolve the reversibly bound surfactant (e.g. oleic acid), (2) maintain conditions for binding of the linker to the inorganic core, (3) fluidize the dispersant, e.g. PEG, (4)while keeping the dispersant in a low $R_G$ (low solubility or low coil volume) conformation.

The inventors have investigated the consequences of limits of the grafting-to approach by the state-of-the art method of Park et al. [Park et al., supra], which results in monodisperse spherical particles with a shell of a surfactant. The goal was to obtain a dispersant (e.g. PEG) stably anchored to the inorganic core through the linker. Grafting-to through direct ligand replacement of surfactant with dispersant was compared to first priming the NP surface by modifying it with the linker and then reacting various end-modified dispersants to the linker having a reactive group that can form a bond with the end-modification of the dispersant (Fig. 1 bottom). As depicted in Fig. 1, three different types of reaction environments were tested to minimize the footprint during grafting: (a) reaction in solution; (b) microwave (MW) induced reaction in poor solvent and (c) reaction in pure polymer melt. All selected conditions were suitable to increase the surface density of the dispersant above the previous value achieved by grafting to nanoparticles synthesized by the Park et al. and analogue methods. Some methods even achieved a particular high surface density (1 M/nm$^2$ and more) above the previously achieved density for the polydisperse bare core grafting method (Amstad et al., supra). According to the invention, colloidally stable, monodisperse, spherical core-shell nanoparticles were provided.

The inventive method comprises a two-step modification of the inorganic core surface, which allows optimization of the conditions for linker binding and dispersant grafting to a linker-modified particle surface.

As used herein, "comprising" shall be understood as referring to an open definition, allowing further members of similar or other features. "Consisting of" shall be understood as a closed definition relating to a limited range of features.

In a first step the nanoparticle surface is grafted with an anchor group in form of the linker which covalently or near-covalently or by ionic bond binds to the surface atoms of the inorganic nanoparticle core. The linker carries one or more additional chemical functionalities that can be reacted to (one or more) organic molecules to make up a dispersant shell. Of course more than one type of linker may also be used. A further possibility is connecting linkers when ligated onto the inorganic particle core, such as by cross-linking. One linker (before optional crosslinking) may have one or more such chemical functionalities, e.g. two of the same type or of a different type - e.g. to control two or more different binding reactions with two or more different dispersants. In general, it is also possible to bind more than one dispersant to the linker - even if the linker just has one binding chemical functionality. Then a competitive reaction between the different dispersants will occur if reacted at the same time - subsequent reactions are also possible.

In a preferred embodiment at least one chemical functionality of the linker for binding to the inorganic particle core is a nitrocatechol group with an amine functional group which is grafted to superparamagnetic iron oxide nanoparticles synthesized with an oleic acid surfactant shell through ligand replacement under non-oxidative conditions. Any material or condition can of course be generalized and adapted within the scope of the claimed invention. Magnetic resonance contrast agents having inorganic non-metallic cores including, but not limited to $FeO_x$, FeOOH and $FeC_x$, can be modified with the dispersants of the present invention.

The linker may have a group suitable for forming one or more, preferably at least 2, bond(s) with the inorganic core. The linker may e.g. comprise an alcohol or carboxyl group in contact with the surface of the inorganic core, preferably a moiety selected from a 6 membered homocycle comprising oxygen or hydroxyl substituents such as described in WO2011/026572 A1 (incorporated herein in its entirety), such as benzoic acid, phenol or catechole, and/or preferably wherein the linker molecules comprise an aromatic group with an electronegative group bound thereto, more preferred

a nitro group. An electronegative group may be selected from F, Cl, Br, I, $NO_2$, COOH, OH, $SO_4$, $PO_4$. Further 6 membered homocycles are selected from quinolines, pyridines and/or derivatives thereof. Preferably the linkers are bound irreversibly to the inorganic particle core. Irreversible binding of linkers to the nanoparticle core surface as used herein refers to an adsorption constant $k_{on}$ >> than the desorption constant $k_{off}$ of the linker to the nanoparticle core surface. In particular, a parameter to select in the materials used is the effective $k_{off}$ of the linker to the nanoparticle core surface. It should be sufficiently low to yield insignificant dispersant loss during long-term storage. The anchor groups of the present invention are effective to irreversibly immobilize dispersants via the linker on magnetic nanoparticles, thereby achieving good nanoparticle stability in dilute and high salt aqueous environments up to temperatures above 90°C. Especially preferred linker molecules comprise a moiety selected from nitrocatechol, nitroDOPA or nitrodopamine, 4-nitro-substituted catechol groups and derivatives thereof, such as in particular 6-nitro-DOPA and 6-nitrodopamine.

The linker is preferably ligated to the inorganic particle core in an organic solvent, preferably an aprotic solvent such as DMF. Preferably the ligand has a negatively charged group, e.g. a carboxylic acid, sulfuric acid or phosphoric acid group.

The result is a nanoparticle with the maximum packing density of reactive (e.g. amine) groups on the nanoparticle surface allowed by the steric constraints by the small linker molecule. Such a reactive surface can be prepared by other small linker molecules and the binding chemistry used for the required strong (preferably irreversible) linking to the nanoparticle core surface has to be tuned to the nanoparticle core material.

In the second step the dispersant carrying one reactive group and optional additional functional groups are reacted to bind the linker. In a preferred embodiment, the dispersant is a polymer and the reaction conditions are at an elevated temperature sufficient to create a polymer melt or strongly collapsed polymer solution in a bad solvent of the dispersant. A melt is preferred as a melt has the minimum size per polymer coil, but considerable internal mobility of the chain segments remain and the reactive group remains accessible. The constraints imposed on mobility and reactivity by the non-solvated conditions are solved by performing the reaction at elevated temperatures or using microwave assisted reactions. In the best demonstrated mode, a melt of aldehyde-poly(ethylene glycol) reacts with nitrodopamine coated particles at 110°C in excess polymer. As above, this can of course be generalized to other materials or conditions within the scope of the claims. Thus in preferred embodiments, binding the dispersant to the linker is an aldehyde-amine binding reaction, and/or preferably the dispersant is in a molten state in the dispersant to the linker binding reaction. In preferred embodiments, the invention comprises raising the temperature of the dispersant above its melting temperature and performing binding above the melting temperature.

The examples demonstrate the inventive concepts for a range of different suitable reactions, with preferred reactive groups and conditions for different applications.

The dispersant is preferably a macromolecule providing steric/osmotic colloidal stability in the preferred environment of the application, e.g. a polymer, such as poly(ethylene glycol) (PEG; especially for aqueous, e.g. biomedical, applications) or polyisobutylene (PIB; e.g. in applications as polymer filler materials such as to produce impact resistant polypropylenes). Further polymers with preferred properties, uses and utilities are: polyoxazolines (including different thermoresponsive derivatives, for biomedical applications), poly(N-isopropylacrylamide) (thermoresponsive polymer, for biotechnological applications, separation, responsive membranes and drug delivery capsules), polyisobutylene (in applications as polymer filler materials such as to produce impact resistant polypropylenes), caprolactone (low melting point, biodegradable, biomedical applications), polyimide (very resistant, KEVLAR, filler material impact resistant materials), polythiophene (conductive polymers, smart materials applications), polypropylene/polyethylene (filler materials), polyacrylic acids and other polyelectrolytes (pH and electroresponsive, smart material applications, polyvinylpyrrolidone, polyvinylalcohol (biocompatible, biomedical and biotechnological applications. A macromolecule is a very large molecule commonly, but not necessarily, created by polymerization of smaller subunits. The subunits of the macromolecule or polymer may be homogenous or heterogenous. Preferred dispersants comprise hydrocarbon groups, which encompass any polymers soluble in organic solvents. Typically, "hydrocarbon chains" include linear, branched or dendritic structures. Different forms of hydrocarbon chains may differ in molecular weights, structures or geometries (e.g. branched, linear, forked hydrocarbon chains, multifunctional, and the like). Hydrocarbon chains for use in the present invention may preferably comprise one of the two following structures: substituted or unsubstituted $-(CH_2)_m-$ or $-(CH_2)_n-Het-(CH_2)_o-$, dendrimers of generations 1 to 10 where m is 3 to 5000, n and o are independently from another 1 to 5000 and Het is a heteroatom, wherein the terminal groups and architecture of the overall hydrocarbon chains may vary. E.g. in the final particle there will be an anchor group which is formed by the linker molecule. This description includes any linear or branched hydrocarbon chains with ratios of unsaturated : saturated bonds varying from 0 : 100 to 100 : 0. In some embodiments the hydrophobic spacer comprises e.g. > 50% of subunits that are $-CH_2-$. In alternative or combined embodiments at least 10% of the carbon atoms, e.g. 10% to 50%, more preferred 20% to 40%, of the hydrocarbon chains are substituted by a heteroatom. Heteroatoms may be selected from O, N, S or N, preferably O. Side chain substitutions can be at a C or at Het with the substituents being selected independently from heterosubstituted or non-heterosubstituted, branched or unbranched, saturated or unsaturated hydrocarbons with 1 to 20 atoms, preferably 2 to 10, especially preferred 2 to 6 atoms in length.

The dispersant may have an average mass of 1 kDa to 30 kDa, preferably of 2 kDa to 20 kDa, especially preferred of 3 kDa to 15 kDa or 4 kDa to 10 kDa. Molecular weights down to about 1 kDa for hydrophilic sterically repulsive spacers such as poly(ethylene glycol) (PEG) already provide good results. Preferred, however, are larger spacers resulting in dispersants having molecular weights in the low kDa range, e.g. in the range of 1.5 to 8 kDa, more preferred in the range of 1.5 to 5 kDa. These ranges are especially suitable for medical applications. For other applications higher kDa ranges may be preferred.

The linker on the other hand is usually a small molecule, e.g. with an average atomic mass of 75 g/mol to 1000 g/mol, preferably of 85 g/mol to 700 g/mol, especially preferred of 100 g/mol to 500 g/mol, even more preferred 120 g/mol to 400 g/mol, such as 140 g/mol to 300 g/mol. The small size of the linker allows dense binding to the inorganic core during common reaction conditions, while still maintaining accessibility to the dispersant. As said above more than one linker type, e.g. 2, 3, 4, 5, 6 or more may be used, especially when more than one dispersant shall later be bound to the linkers and each dispersant is bound to a certain linker binding functionality as mentioned above. It is usually better controllable to bind different linkers at the same time than different dispersants, which may vary in optimal binding conditions.

After binding the linker to the inorganic core, the dispersant and the linker are reacted to form a bond. To this end, the linker and the dispersant each comprises one member of a pair of reactive groups, capable of forming a chemical bond in the ligation reaction. Example pairs of reactive groups are an amine and an aldehyde, an amine and an acrylate, an amine and a carboxyl group, an amine and an ester group. Preferably the amine is a primary amine. Further pairs are constituted by a sulfonylchloride and either an amine or a hydroxy-group, an acid-chloride and either an amine or a hydroxy-group, a thiol and any one selected from a maleimide, an acrylate or an allyl-group, an isocyanate and either a hydroxy-group or an amine, a conjugated dien and a substituated alkene (for Diels-Alder-reaction), an epoxide and a nucleophile (such as alkyl-halogenides, alcoxides, amines). Especially preferred is an amine and an aldehyde that are reacted in the binding reaction, thereby forming an imine. Even more preferred, the imine is reduced to an (secondary) amine by a reducing agent, such as $NaCNBH_3$. The less reactive group of the pair, such as the amine, is preferably on the linker to prevent any off-reactions of the more reactive group, e.g. an aldehyde, that is then preferred on the dispersant. It may also be the other way around. Preferably binding the linker to the dispersant is a reaction without the requirement of a solvent. Preferably a solvent is absent during the reaction or only in small amount, e.g. less than 50% (w/v), or less than 20% (w/v). The reaction is preferably between reactive groups that do not require a solvent, that might in other cases be required to stabilize or participate in a transition state. The inventive groups are preferably selected to only require contacting of the pair of functionalities for the reaction to occur. Leaving groups may be present though.

The linker or dispersant may comprise a leaving group that is removed in the ligation reaction. Leaving groups can be halides such as $Cl^-$, $Br^-$, and $I^-$, sulfonate esters, such as tosylate ($TsO^-$) or mesylates, succinates or succinimid esters, perfluoro-alkylsulfonates, water or ammonia, inorganic esters such as phosphates or nitrates, thiolates, alcohols.

Further binding reaction may involve an addition, such as by an alkene or alkine group, preferably by an acrylate.

Using dispersants of the present invention it is also possible to reduce non-specific adsorption of the particles to other molecules, in particular biomolecules such as, but not limited to, proteins, peptides, and thus to increase e.g. good half-life time of the particles in biological fluids. This may be achieved by providing particles with stealth dispersants, e.g., but not limited to, poly(ethylene glycol) and poly(methyloxazoline) covalently linked to linker groups as defined herein. This effect is due to the high dispersant packing densities that can be achieved with such dispersants having linear or branched, also comprising dendritic or hyperbranched, spacing groups or a combination of such spacing groups.

In the inventive method, in preparation of the binding reaction of the dispersant to the linker, the concentration of the dispersant can be above the solubility concentration of a solvent whereby the dispersant is not fully dissolved, thereby obtaining a suspension and wherein the dispersant is fluidized by increasing the temperature above the melting temperature of the suspension. This will provide a fluidized dispersant at a high concentration and high temperature that is suitable to achieve the high surface densities.

In other embodiments, the dispersant may be provided in solution at ambient temperatures, e.g. at between 10°C to 40°C. In other embodiments the dispersant may be provided as a melt without a solvent. Although any temperature in the range of 10°C to 200°C is suitable, high temperatures are preferred.

Preferably, binding the dispersant to the linker molecules is performed at a temperature of at least 80°C, preferably of 85 °C to 150°C. In preferred embodiments heating is assisted by microwave irradiation, especially microwave irradiation during the binding reaction, which will further assist the reaction in addition to the high temperature effects.

With the inventive method, high surface densities of bound dispersant molecules can be achieved, e.g. at least 1.1, preferably at least 1.2, even more preferred at least 1.3, at least 1.4, at least 1.5, at least 2, at least 2.5, at least 2.8, at least 2.9, at least 3, at least 3.1, at least 3.2, at least 3.3 or at least 3.4, dispersant molecules per $nm^2$ of the inorganic core surface. As said above with regard to method effects being related to product characteristics, such densities may also define the inventive particles or preparations thereof. Such particles can be selected from the particles obtained by the inventive method. These densities are preferably determined by TGA (thermogravimetric analysis), especially as shown in example 7.2 using air or an oxygen containing gas.

In further preferments of all embodiments of the present invention the step of providing an inorganic particle or particle cores comprises providing a plurality of inorganic particles wherein the mean standard deviation of the particle's average size is at most 10%, preferably at most 5%, even more preferred at most 2% of the particle's average size, such as at most 0.8 nm, preferably at most 0.5 nm. Such particles may be synthesized as is with the method described above (e.g. the cores provided without size separation) or selected after size separation.

The standard deviation (SD) measures the amount of variation or dispersion from the average. The standard deviation of size distribution is the square root of its variance.

A "plurality" as used herein refers to several particles, which may differ within certain parameter thresholds in parameters such as size. The amount of the particles can be at least 100, at least 1000, at least 10000, at least 100000, at least 1 Mio., at least 10 Mio. etc.. Preferred ranges are e.g. 100 to 100 Mio.

A preparation of such a plurality of particles can e.g. be defined as a preparation, wherein

- said particles comprise an inorganic core surrounded by metal complexing linkers that are chemically linked to dispersant molecules,
- wherein the dispersant molecules (a) are at an average density of at least 1.1 dispersant molecules per $nm^2$ of the inorganic core surface, or any one as said above, and/or (b) form a shell of constant dispersant density and a further shell of gradually reduced dispersant density with increasing distance from the inorganic core surface. The shell of constant dispersant density is an inner shell and the shell of decreasing density (with the radius or distance) is an outer shell. The inner shell can also be defined as a shell of similar density to the molten polymer state of the dispersant identifiable by small angle x-ray scattering in a solvent, such as water for hydrophilic dispersants, and the outer shell can also be defined as a solvable shell, distinct from the dense inner polymer shell. This shell structure forms is solution when the outer shell may e.g. form an outreaching or brush like structure, usually heavily solvated, while the inner shell remains dense, possibly not or only residually solvated without influencing its dense structure. The solvent for the small angle x-ray scattering determination and for establishing the two-shell like structure should be a good solvent for the dispersant, e.g. to lead to a solvation of the dispersant in the outer shell and not to a collapsing thereof. "Good solvent" relates to the excluded volume parameter of the dispersant under these conditions. E.g. a good solvent for a hydrophilic dispersant is a hydrophilic solvent and a hydrophobic solvent for a hydrophobic dispersant.

Preferably the inorganic core is of an average size between 2 nm to 80 nm in diameter, or any one as said above. The particles of the preparation can be of homogenic size in said plurality wherein the mean standard deviation of said average size is at most 10%, preferably at most 5%, even more preferred at most 2% of said average size, such as at most 0.8 nm, preferably at most 0.5 nm. This preparation can be obtainable by a method of the invention.

Further provided is a particle comprising an inorganic core surrounded by metal complexing linkers that are chemically linked to dispersant molecules, wherein the dispersant molecules (a) are at an average density of at least 1.1, preferably at least 3.0, dispersant molecules per $nm^2$ of the inorganic core surface (or any number as said above), and/or (b) form a shell of constant dispersant density and a further shell of gradually reduced dispersant density with increasing distance from the inorganic core surface or form a shell of similar density to the molten polymer state identifiable by small angle x-ray scattering in a solvent, e.g. water, and a solvable shell, distinct from the dense inner polymer shell - as said above for the particles of the preparation. This particle can be obtainable by a method of the invention.

The inventive particles having the high dispersant surface density surprisingly retain a shell having a molten state like appearance, illustrating the high density of this shell portion. Outside of this dense inner part of the shell where the density is similar to that of the molten polymer state, the dispersants have a similar structure as in previously reported particles (Amstad et al., supra, WO2011/026572 A1), e.g. in the case of PEG a spherical brush like structure, wherein the density decreases monotonously with distance from the particle core. In the dense molten state like shell the density of the dispersants is constant at maximum density in the entire distance range of this shell or shell portion from the core. This molten state shell is unique to the inventive particles illustrating the difference in density to previous particles - although and as discussed above Amstad et al., supra, WO2011/026572 A1 reported high surface densities which was an artefact of calculation of surface area based on a spherical particle imprecision. This molten state shell of the inventive particles is visible in small angle x-ray scattering in water, e.g. as shown in example 10, and can be detected by this method. Preferably the molten state shell is calculated from X-ray data by the Daoud-Cotton model (as demonstrated in example 10). The molten state like shell is preferably at least 0.3 nm thick, especially preferred at least 0.5 nm, at least 1 nm or at least 2 nm. In the molten state like shell solvents may be absent or at very low concentrations due to infiltrations. Solvent amounts are preferably below 40 % (w/v), especially preferred below 20% (w/v) or below 10 % (w/v). Such a solvent may be water in case of hydrophilic dispersants like PEG, polyoxazoline or PNIPAM.

The inventive particles or preparations, e.g. as produced by the inventive method, can be used for various applications, including biomedical contrast agents, multifunctional nanoparticles for biomedical applications, nanoparticle building blocks for assembly of smart materials, therapeutic magnetic nanoparticles, magnetic labeling and separation.

Such methods may comprise detection the inventive particles in a sample or separating the inventive particles within or from a sample volume, e.g. by applying a magnetic field and ordering the magnetic particles of the invention within such a field. A sample may be fluid comprising a complex mixture of various components. Further, such particles can be filler materials, stiffeners, or additives for improved conductivity, magnetic responsiveness or optical properties in polymer material applications.

The present invention is further illustrated by the following examples without being limited to these embodiments of the present invention.

**Figures:**

**Fig 1.** Schematic representation of the direct ligand exchange method (top) and the two-step "grafting-to" synthetic procedure (down) for the synthesis of core-shell- nanoparticles.

**Fig. 2.** Transmission electron microscopy (TEM) images of 7.9± 0.4 nm large iron oxide nanoparticles (oleic acid ligands). As can be seen at higher magnifications (right image) the particles are monocrystalline.

**Fig. 3.** Exemplary depiction of the column purification (25cm Sephadex G75 superfine in Milli-Q water) results of a typical two-step synthesis (ALD-melt; Table 1, entry 15). Fractions were collected (x-axis) every 5 mL. The yield after freeze-drying (left y-axis) and the grafting density (right y-axis) are shown for every fraction. The yields are calculated on the basis of pure $Fe_3O_4$ cores (inorganic fraction); Fr. 6 consists of pure PEG molecules (mass of organic weight: 99.8%), therefore the calculated grafting density is out of the shown range.

**Fig. 4.** NMR-spectra of nitro-dopamine (1). Due to the high water content of the NMR-solvent the primary amine and the two hydroxyl-groups cannot be seen.

**Fig. 5.** Typical TEM image of the particles of the invention.

**Fig. 6.** Pebbles results of a TEM image. (a) TEM image as measured (b) pebbles automatically identifies and marks the nanoparticles and provides (c) the statistical evaluation of the size distribution of the particles in the respective TEM-image given by Pebbles.

**Fig. 7.** TEM-pictures of main fractions of an ALD-melt (Table 1, entry 15). (a) fraction I shows few aggregates with small particle-particle distances; (b) Fraction III (main fraction) show no aggregation, no free PEG whereas in (c) (fraction V) one can immediately identify huge areas of free (unbound) PEG

**Fig. 8.** Example for the TGA-curves of the main fraction (here: ALD-melt, table 1, entry 15)

**Fig. 9.** Representation of the Daoud-Cotton blob model for a spherical brush with a blob radius $\overline{\xi(r)}$ of an individual blob. The transition radius between melt-like and unswollen behavior is indicated by r1=r2.

**Fig. 10.** Radial electron density distribution (corresponding to polymer density) in the core region (blue, left horizontal) predicted by the Daoud-Cotton model, the constant density region (melt like region) (purple, middle horizontal) and the $r^{-4/3}$ decay region (brown, right). For reasons of visibility the ΔSLD is increased to 0.5.

**Fig. 11.** SAXS from the cores (red, squares) and the fitted function (black), inset showing TEM images of the particles, scalebar 10 nm.

**Fig. 12.** Squares: SAXS from a core-shell particle with a distinct shoulder from the shell (0.1 $A^{-1}$) and the scattering from the cores in the range from 0.23 to 0.42 $A^{-1}$. SAXS from core-shell particles with different concentrations (black squares, 5 mg/ml; red circles, 1 mg/ml and blue triangles, 0.5 mg/ml).

**Fig. 13.** Scattering curve of the Core-Shell particle in MQ and the respective fit function as obtained by the core-chell density model built on the Daoud-Cotton blob model.

**Fig. 14.** Cyclic change of temperature of core-shell particles from 25°C (green circles) to 50°C (red triangles) and subsequent cooling to 25°C (cyan squares) and the respective fitted functions

**Fig. 15.** Normalized fit functions for the shell behavior in cloudpoint buffer in the q region of the shell for 25°C (green, top at y-axis) to 50°C (red, bottom at y-axis) and subsequent cooling to 25°C (cyan, middle at y-axis). The inset shows the complete q range.

**Fig. 16.** Left: Nanoparticles from the samples of Amstad et al. 2009, supra, reported to have 2.8 $M/nm^2$ grafting density using a direct grafting method. Right: Nanoparticles having the same or higher grafting density using the two-step melt grafting method of the invention, trimmed down to a maximum <1 $M/nm^2$ grafting density using the direct grafting method. Although both particles are similar, have the same PEG shell, behave identically and have about the same dispersant density determined by e.g. SAXS, the determined density values by TGA differ due to overrepresentation in the previous work.

**Examples:**

**Example 1: Reagents and materials**

All used chemicals were purchased at Sigma-Aldrich and used without further purification.

**PEG-ALD:** O-[2-(6-Oxocaproylamino)ethyl]-O'-methylpolyethylene glycol 5'000 ("PEG-aldehyde 5000");
**PEG-ACRY:** Poly(ethylene glycol) methyl ether acrylate;
**PEG-TOS:** Poly(ethylene glycol) methyl ether tosylate;
**PEG-NHS:** Methoxypolyethylene glycol 5,000 acetic acid N-succinimidyl ester; purchased from JenKem-USA (Mn~5000; PDI=1.1; functionality of the endgroup >95%)
Carl Roth dialyzing membranes (regenerated cellulose) with a cut-off size of 5000 Da were used to purify the nitrodopamine modified NPs.

**Example 2: Synthesis of nitro-dopamine and nitro-dopamine-PEG(5000)**

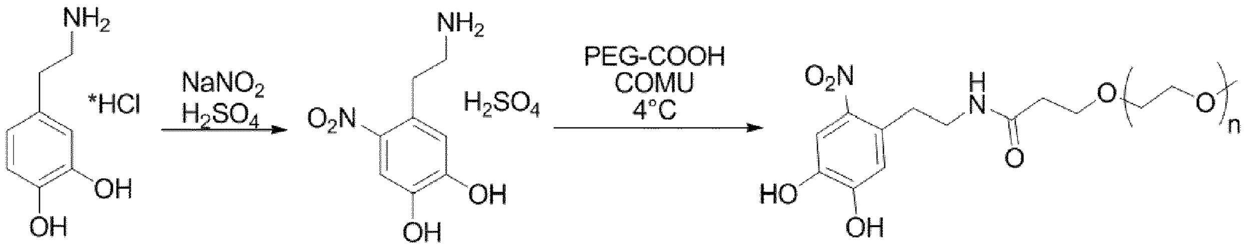


1 2

**Scheme 1:** Synthetic path way for the synthesis of nitrodopamine and the NHS-activated coupling to the PEG5000 using a COMU® ((1-Cyano-2-ethoxy-2-oxoethylidenaminooxy)dimethylamino-morpholino-carbenium hexafluorophosphate)-activated intermediate step. Nitro-dopamine (1) can be used in the two-step method

**2.1 Synthesis of nitro-dopamine.** Dopamine hydrochloride (20 g; 105 mmol) was dissolved in 600 mL water, sodium nitrite (25 g, 362 mmol) was added and the mixture was cooled to 0°C using an ice bath. 200 mL of a 20 v/v% sulfuric acid was slowly dropped into the reaction. After removing the ice-bath the mixture was stirred at room temperature overnight. The crude product was collected by filtering the obtained dispersion. After washing the product with ice-cold water the nitrodopamine hydrogensulfate was dried in high vacuum and stored at 4°C until further use. NMR spectra of n-dopamine is shown in Fig. 4. Also analysed was pure nitrodopamine; a mass peak at 199.10 could be correlated to the nitrodopamine (m=198.06+1 (proton)=199.06). Higher mass-peaks could be assigned to polymeric structures of nitro-dopamine and assoziatives with the matrix materials

**2.2 Synthesis of MeO-PEG-nitro-dopamine.** 1 g MeO-PEG-COOH (0.2 mmol) was dissolved in 5 mL DMF (headspace grade 99.99%, anhydrous) and cooled to 4°C on an ice bath. COMU® (95 mg; 0.22 mmol) and N-methylmorpholine (0.24 mL; 2.2 mmol) were added and the mixture was stirred for 2 hours. Afterwards a solution of nitrodopamine hydrogensulfate (53 mg; 0.18 mmol) in DMF (0.5 mL; 99.99%) was dropped in during 5 minutes. The reaction was stirred for 2 h at 4°C and 16 h at RT. The solution was poured into 45 mL ice cold acetone and centrifuged (5000 rpm, 4°C, 10 minutes). The precipitate was washed 2 times with acetone and afterwards dissolved in 1 mL water. The crude product was dialyzed against Milli-Q-water for 48 hours. Freeze drying gave 1 g (0.19 mmol) of pure product (0.95 g; yield 95%).

**Example 3: Synthesis of monodisperse $Fe_3O_4$ nanoparticles.**

To prepare monodisperse iron oxide nanoparticles [S.-J. Park et al., J. Am. Chem. Soc. 2000, 122, 8581-8582; T. Hyeon et al., Journal of the American Chemical Society 2001, 123, 12798-12801; E. Kang et al., Journal of Physical

Chemistry B 2004, 108, 13932-13935; J. Park, et al., Nat. Mater. 2004, 3, 891-895; J. Park, et al., Angew Chem Int Ed Engl 2005, 44, 2873-2877; S. G. Kwon et al., J. Am. Chem. Soc. 2007, 129, 12571-12584; J. Park et al., Angew. Chem., Int. Ed. 2007, 46, 4630-4660; C. Cavelius et al., Crystal Growth and Design 2012, 12, 5948-5955.], $Fe(CO)_5$ (1 mL; 1.49 g; 7.4 mmol) was added to a mixture containing 25 mL of dioctylether and 3 mL oleic acid (10 mmol) at 100°C. The resulting solution is heated to 290°C with a heating rate of 10 K/min (reflux) and kept at that temperature for 1 hour. To gain full reproducibility and control over the temperature during the nanoparticle synthesis a thermo controller LTR 3500/S from Juch-heim-Solingen was used. During this time the color changes from the initial orange to dark black. After cooling the mixture to room temperature the crude product was poured into 175 mL nitrogen-bubbled acetone. After centrifugation (5000 rpm, 10 min, 20°C) the black product was redispersed in a small amount of toluene (~1 mL) and reprecipitated in acetone (190 mL). This centrifugation-redispersion-precipitation step was repeated 3 times overall. The resulting oleic acid protected iron oxide nanoparticles were used immediately for further reactions. The size of the particles could be controlled by the ratio of the iron pentacarbonyl and the oleic acid as well as the heating rate during the synthesis. Particles with 3.9±0.3 nm to 4.4±3 nm have been used.

**Example 4: Ligand-exchange with nitrodopamine-PEG5000-OMe (one-step-method)**.

The oleic acid-covered nanoparticles (25 mg) were dispersed in 20 mL DMF (headspace grade 99.99%, anhydrous) and nitrodopamine-PEG5000 (example 2.2) (500mg; 1.7 mmol) was added. This solution was purged with nitrogen for 2 minutes, put in an ultrasonic bath for 60 minutes, allowed to stand at room temperature for 15 hours, ultrasonicated again for 60 minutes and precipitated in cold acetone (180 mL). After centrifugation and 5 washing steps (redispersion in methanol - centrifugation with 5000 rpm, 10 minutes, 20°C) the obtained core-shell iron oxide NPs (FeOx-PEG) were characterized using TEM, TGA, DLS.

**Example 5: Core-shell-nanoparticles via two-step grafting-to reaction**

**5.1 Ligand-exchange (oleic acid to nitrodopamine).** The oleic acid-covered nanoparticles (~3 g - whole amount of previous synthesis) were dispersed in 20 mL DMF (headspace grade 99.99%, anhydrous) and nitrodopamine hydrogensulfate (n-dopamine) (150 mg; 0.5 mmol) was added. This solution was purged with nitrogen for 2 minutes, put in an ultrasonic bath for 60 minutes, allowed to stand at room temperature for 15 hours, ultrasonicated again for 60 minutes and precipitated in pure acetone (180 mL, cold). After centrifugation and 5 washing steps (redispersion in methanol - centrifugation with 5000 rpm, 10 minutes, 20°C) the obtained nitro-dopamine modified iron oxide NPs ($NP-NH_2$) were used directly for grafting with PEG.

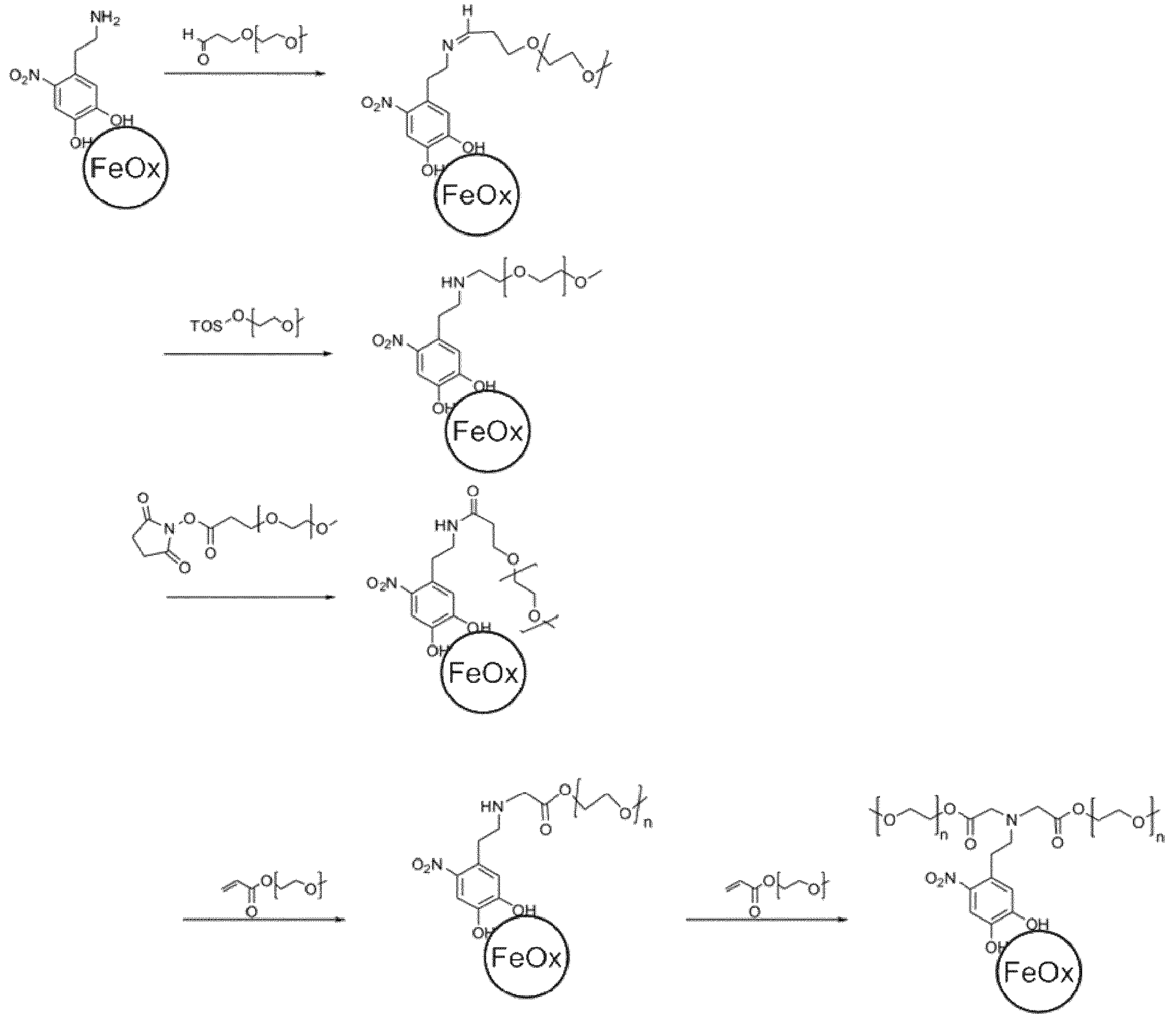

**Scheme 2:** Schematic representation of the different grafting-to reactions. Aldehyde reacts under condensation forming an imine, PEG-tosylate with nucleophilic-substitution to form a secondary amine, the NHS-activated coupling leads to a stable amide-bond and acrylates undergo a Michael-addition to form secondary or tertiary amines.

**5.2 Synthesis of PEG-core-shell nanoparticles using grafting-to reaction in solution.** The as-synthesized nitrodopamine nanoparticles (50 mg) were dissolved in dry DMF (5 mL, headspace grade 99.99%, anhydrous), the respective PEG-derivate (500 mg, 1.7 mmol, NHS, TOS, ALD) was added and the mixture was stirred at room temperature for 16 hours. After pouring the reaction mixture into 195 mL cold acetone and centrifugation (5000 rpm, 10 minutes, 4°C) the precipitate was dissolved in water (2 mL), filtered through a 0.45 μm syringe filter to remove aggregates and purified as described below.

**5.3 Synthesis of PEG-core-shell nanoparticles using microwave assisted reactions.** The as-synthesized nitrodopamine nanoparticles (50 mg) were dissolved in dry methanol (3 mL) the respective PEG-derivate (500 mg; ~1.7 mmol; NHS; ACRY; TOS; ALD) was added and this mixture was heated in a CEM lab microwave to 140°C for 90 minutes. After cooling to room temperature the reaction mixture was poured into 190 mL cold acetone and centrifuged for 10 minutes with 5000 rpm (4°C). The precipitate was dissolved in water (2 mL), filtered through a 0.45μm syringe filter to remove aggregates and purified as described below.

**5.4 Synthesis of PEG-core-shell nanoparticles using polymer melts.** The freshly synthesized nitrodopamine

modified nanoparticles (~50 mg) were roughly mixed with the dry PEG-derivate (TOS, NHS, ALD) (500 mg; 1.7 mmol), purged with nitrogen and slowly heated to 110°C. After the material is completely molten a nitrogen-flow (needle) is used for mixing the melt continuously. The reaction was kept at this temperature for 90 minutes. After cooling to room temperature the black solid was dissolved in water (2 mL) (ultrasonic bath for 1 hour), filtered through a 0.45 μm syringe filter (RC) to remove aggregates and purified as described below.

**Example 6: Purification of PEG-iron oxide core-shell nanoparticles.**

The dark brown/black solution is applied onto a 25 cm long (3 cm diameter) column filled with Sephadex G75 (milli-Q water). Pure water was used for the purification. Fractions of 5 mL were collected, after filtering the single fractions through 0.45 μm syringe-filters and freeze drying the products were characterized using TEM, TGA and DLS.

**Example 7: Characterization of core-shell nanoparticles**

**7.1 Characterization of the core-shell nanoparticles using TEM.**

A small amount of product was dissolved in water (nitrodopamine and PEG-modified particles) or toluene (oleic acid modified nanoparticles) and dropped onto the TEM-grid (3.05 mm HR-TEM-grid, copper 300 mesh, carbon film, Gröpl Austria). All pictures were taken using a FEI TECNAI at 200kV. A typical TEM image is shown in Fig. 5.

The statistical size distribution was obtained by the use of the freeware Pebbles (http://pebbles.istm.cnr.it). Pebbles results of a TEM image: (a) TEM image as measured (b) pebbles automatically identifies and marks the nanoparticles and provides (c) the statistical evaluation of the size distribution of the particles in the respective TEM-image given by Pebbles. TEM-pictures of main fractions of an ALD-melt are shown in Fig. 7.

**7.2 Characterization of the core-shell nanoparticles using TGA.**

1-3 mg of the respective sample were weighed into 70μl $AlO_x$-cups and measured on a Mettler-Toledo TGA/DSC 1. The samples were measured in a constant flow of synthetic air (80 mL/min plus 20 mL nitrogen stream as protection gas for the balance) with a heating rate of 10 K/min. Analysis was performed using the Mettler-Toledo software (simple step-function from 150-400 °C).

A TGA-curve is shown in Fig. 8 for an ALD-melt. The grafting density and parameters for the ALD melt are:

***ALD-melt:***

organic content: 88.6%
r= 2.0 nm
M= 5200 g/mol
Density(PEG)= 1.12g/$cm^3$
<u>Density (ironoxide)= 5.24 g/$cm^3$</u>
V(NP)=33.5 $nm^3$

<u>Mass (1 NP)= $1.8\times10^{-19}$ g/NP (core)</u>

0.885 g PEG per gram product
$1.7\times10^{-7}$ mol PEG
$1.0\times10^{17}$ molecules PEG
0.115 mg ironoxide per gram product
<u>$6.5\times10^{14}$ NP/g</u>
3.1 molecules PEG /$nm^2$

**7.3 Characterization of the core-shell nanoparticles using DLS.**

The count-rate and hydrodynamic size of core-shell nanoparticles redispersed in Milli-Q water were measured before and after temperature cycling (20-90 °C) to assess the colloidal stability and the stability of the surface coating

[E. Amstad et al., Nano Letters 2009, 9, 4042-4048.].

**Example 8: Purification and characterization of core-shell ironoxide-nanoparticles**

All PEGylated samples were dissolved in a small amount of water (2 mL of water per g of raw product) and added onto a 20-25cm long column of Sephadex G75 superfine (3cm diameter). During the separation process a high percentage of the nanoparticles stick to the Sephadex material - this lost fraction is considered as the unmodified (nitrodopamine-modified) and aggregated parts of the raw product (See TEM-analytics of single fractions). The fractions were collected manually, filtered through a 0.45μm syringe filter and freeze dryed to obtain the products.

**Example 9: Results and best mode**

The NPs were synthesized by an optimized heat-up process leading to $Fe_3O_4$ NPs (a) with well-defined size (adjustable between 3.5-9 nm in diameter; PDI≤1.05); (b) highly spherical and (c) monocrystalline (see figure 2). All grafting experiments were done with particles in the size range between 3.8±0.3 to 4.2±0.3 nm in diameter NPs to exclude size or curvature effects.

All grafting methods were performed with an excess of free polymer to ensure that the maximum possible grafting density could be achieved. A key step to characterize polymer-grafted core-shell NPs is the removal of residual or weakly bound polymer from the sample. Previous works indicate that magnetic decantation, dialysis and centrifugation all lead to substantial amounts of residual PEG even after multiple applications, while size exclusion chromatography can separate free PEG from grafted particles. Dextran column chromatography can also be used to evaluate the colloidal stability since too sparsely grafted NPs have high affinity for dextran chromatography columns; insufficiently densely grafted particles therefore aggregate to the column material. All samples were therefore purified using a 20-25 cm long and 3 cm broad Sephadex G75 filled column. This length was chosen as it generated several distinguishable fractions for further analysis. Fractions were separated every 5mL. Samples obtained by the direct ligand exchange of oleic acid for nitrodopamine-PEG did not pass columns longer than a few cm. These samples were therefore instead purified using multiple centrifugation in methanol/acetone mixtures and using magnetic extraction to get rid of the excess free PEG; residual PEG could be present in the samples even after purification leading to overestimation of the grafting density and was therefore thoroughly removed.

The success of the grafting methods can be compared on a set of dependent criteria such as grafting density, resulting colloidal stability, cost and ease/time of synthesis. The colloidal stability, and therefore the suitability for biomedical applications, is believed to strongly relate to stable and high density of PEG grafting; thus, the grafting density was our primary concern and was determined by TGA for each grafting method and fraction.

Previously, direct ligand exchange of the oleic acid with the anchor-group modified PEG has been used; such ligand replacement requires solvents, e.g. DMF, that strike a compromise between the solubility and $R_G$ of the PEG and oleic acid. The resulting expansion of the PEG coil size above its minimum limits the highest obtainable PEG-molecule grafting density (table 1, entry 2). The achieved density is lower than required for column purification and lower than previously reported to be necessary to achieve non-aggregating particles under high salt, protein or temperature conditions, although due to very different purification and testing conditions the literature is not conclusive on what minimal grafting density is required.

**Table 1.** Overview of the calculated grafting densities of PEG(5kD) obtained by different grafting-to reactions evaluated by TGA after Sephadex G75 column purification.

| Entry | reactant | Method | Grafting density [M/nm2] |
|---|---|---|---|
| 1 | $FeO_X$-oleic acid | | 17.4±5 [f] |
| ***one step grafting-to*** | | | |
| 2 | n-Dopamine-PEG5000 Solution (DMF) | | 0.5±0.2[d] |
| ***two step grafting-to procedure*** | | | |
| 3 | $Fe_3O_4$-n-Dopamine (step 1) | | 3.8±1.2 |
| 4 | PEG-NHS | Solution [a] | 0.9±0.2 |
| 5 | PEG-TOS | Solution [a] | 0.8±1.1 |
| 6 | PEG-ACRY | Solution [a] | [d] |
| 7 | PEG-ALD | Solution [a] | 0.8±0.3; 1.2±0.6[e] |
| 8 | PEG-NHS | Microwave [b] | 1.0±0.3 |

(continued)

| Entry | reactant | Method | Grafting density [M/nm2] |
|---|---|---|---|
| 9 | PEG-TOS | Microwave [b] | 1.6±0.8 |
| 10 | PEG-ACRY | Microwave [b] | 1.3±1 |
| 11 | PEG-ALD | Microwave [b] | [d] |
| 12 | PEG-NHS | melt [c] | [d] |
| 13 | PEG-TOS | melt [c] | 1.1±0.5 |
| 14 | PEG-ACRY | melt [c] | 2.2±0.4 |
| 15 | PEG-ALD | melt [c] | 3.1±0.9 |

[a] 50 mg Fe3O4-n-dopamine particles, 500 mg PEG-X, 3 mL DMF, 16 h at RT; [b] 50 mg Fe3O4-n-dopamine particles, 500 mg PEG-X, 3 mL MeOH, 90 min at 120°C; [c] 50 mg Fe3O4-n-dopamine particles, 500 mg PEG-X, N2-stream, 90 min at 110 °C; [d] sample did not pass the sephadex column, sample purified by 3 precipitation steps in acetone; [e] yield <10%, higher grafting densities and yields could be obtained by adding NaCNBH3 as reduction agent; [f] large amount of free (unbound) oleic acid present

To increase the maximal density of surface grafted polymer it is necessary to change to a two-step grafting approach. First, the oleic acid is replaced completely by nitrodopamine. This reaction can be carried out in an ideal solvent for oleic acid and nitro-dopamine, e.g. DMF, and results in ~4 M/$nm^2$ (table 1, entry 3).

Several different methods for binding end-group modified PEG to the free amine-group of the densely nitro-dopamine modified iron oxide NPs can be envisioned, of which we choose to test: a) the tosylate-group reacting with primary amines under a $S_N2$ reaction; b) the acrylate-group enabling a Michael-addition; c) the aldehyde-group enabling condensation (Mannich-reaction); and d) NHS activated PEG enables the stable formation of an amide. Our goal was to obtain the highest PEG grafting density; given the applications, yields as low as <10% of the $Fe_3O_4$ cores after column purification are therefore acceptable. For polydisperse and amorphously shaped NPs we have previously shown that a PEG(5kD) grafting density of >2 chains/$nm^2$ nonmonodisperse, nonspherical nanoparticles is required for short Sephadex column purification [Amstad et al., 2009, supra]. 25cm long Sephadex G75 column made it possible to separate and analyze several fractions to identify the highest grafting densities and their relative yields. Fig. 3 shows an example of such fractionations of an ALD-melt modification (Table 1, entry 15). A high grafting density fraction (Fr. 3) with 5-10% yield was selected as the product fraction. Before the main peak aggregated cores with <0.2 chains/$nm^2$ were collected. After the product fraction free PEG was collected. The fraction with the highest average ligand density measured by TGA after column purification for three repetitions of each synthetic protocol is given in Table 1.

Table 1 shows that the coupling of NHS-PEG to the nitrodopamine modified particles in DMF slightly improves the grafting density over direct ligand displacement to ~1 chain/$nm^2$, which is sufficient to enable column purification. The other reactive end-group PEGs were also attempted in DMF and resulted in similar grafting densities.

The same reactions were conducted in a MW reactor (Table 1, entry 8-11). The MW-induced high heating of the NP-surface could speed up the grafting-to reactions to enable performing them at much lower solvation using MeOH; by this, the foot print of the PEG is reduced and the grafting density potentially increased. However, the MW-assisted reactions only insignificantly improved the PEG density.

The main limitation of the above strategies is that the footprint of the PEG-coil during grafting is low but not minimized. The reaction efficiency can be increased at the same time as the polymer footprint is minimized by choosing a reaction that performs at an elevated temperature at which PEG(5kD) is in a melt and therefore retains chains mobility. The best fractions using this melt grafting method at 110°C (Table 1, entries 12-15) far surpass the other methods in terms of grafting density. Grafting densities of 2-3 chains/$nm^2$ were consistently achieved, which is only slightly below, 82% of, the initial ~4 nitrodopamine/$nm^2$. This is at least a factor of 2 higher than for similar yields of the other standard grafting-to methods. Given different purification and measurement methods employed in previous studies it is difficult to compare NP grafting densities in absolute terms; however, these densities are higher than the highest reported for grafting-to onto shape and size polydisperse bare $Fe_3O_4$ NPs of ~2.5 chains/$nm^2$ [Amstad et al., 2009, supra]. The amorphous shape in this work leads to underestimation of the true surface area and increases surface accessibility; this can explain the high obtained grafting density compared to our control under the same conditions. As we directly and consistently compare the solvent, MW and melt grafting-to approaches, we can clearly state that the latter is superior in a two-step grafting-to approach by at least a factor of 2 and to ligand replacement by a factor >4, which we could not quantitate due to that the ligand replacement particles did not pass the column used for purification.

The highly grafted monodisperse NPs demonstrated remarkable redispersion compared to previously observed samples with lower grafting density. The powder hydrated and redispersed directly upon addition of smallest amounts of water, indicating that the cores even in the dried state do not come in close proximity to each other. The supreme

colloidal stability was further demonstrated by high stability in water over prolonged period of time (1 year) and by the possibility to repeatedly filter through a 0.45μm syringe filter without loss of material. The absence of aggregation under temperature cycling, which produces aggregation of reversible and/or sparsely grafted NPs, was investigated by filtering through a 0.45μm syringe filter at a successive row of temperatures (25→75→25°C; 10°C steps). As expected the ALD-melt grafted particles were in all tests superior to the lower grafted particles by showing no aggregation or loss of material, whereas all the other grafting methods led to residues in the syringe filter.

For the overall rating of the performances of different grafting methods in addition to the grafting densities, the yields have to be taken into account. Although the microwave assisted PEG-TOS grafting to reaction (Table 1, entry 9) and PEG-ACRY melt reaction (table 1, entry 14) also lead to very high grafting densities (1.6 and 2.2 M/$nm^2$ respectively) the yields were very low in both cases (<3% calculated on the basis of ironoxide cores).

In summary, we have introduced a melt-based approach to densely graft sterically stabilizing shells to mono-disperse, spherical superparamagnetic $Fe_3O_4$ NPs making them suitable for e.g. biomedical applications. Achieving colloidal stability of such NPs which require ligand replacement has hitherto been a bottleneck. A two-step grafting-to method from pure melt of ALD-PEG offered a superior and straightforward alternative to achieve this at sufficient yield (up to 10%). The obtained grafting density and corresponding colloidal properties significantly surpass those of previously presented methods. By approaching grafting of the maximum density of possible grafting sites, a brush density profile of the shell of the core-shell nanoparticles with unique structure (referred herein as "molten like state") and unique properties is obtained.

**Example 10: Analysis of dispersant shell by small angle x-ray scattering (SAXS)**

*10.1 Sample preparation and SAXS measurements*

The purified (example 8) Core-ALD-Shell particles were diluted in MQ water with a known concentration of 5 mg/ml. For the dilution series, concentrations of 5, 1 and 0.5 mg/ml were prepared from the stock solution. The cloud point buffer measurements were carried out in a solution containing 0.5 M NaCl, 0.5 M K2SO4 and 0.08 M MOPS with a core-shell particle concentration of 5.43 mg/ml with temperature steps of 25, 40, 50 and 60 °C. For the SAXS experiments the solutions were prepared in type 0500 glass capillaries supplied by Hilgenberg with a nominal diameter of 1 mm and a wall thickness of 10 μm. They were flame-sealed to rule out contamination and finished with a droplet of epoxy resin to avoid any evaporation during the course of measurement.

The $Fe_3O_4$ cores were prepared as a dry powder between two layers of Scotch tape and measured for 60 s.

Measurements were carried out using a Rigaku S-Max 3000 SAXS system equipped with a copper-target micro focus X-ray tube Mi-croMax-002+ (45kV, 0.88mA), collimated through three pinholes (400, 200 and 700 μm) to achieve a beam diameter at the sample position of 280 μm (FWHM) and a Triton 200 2D multi wire gas-filled x-ray detector (200 mm diameter of active area, spatial resolution 200 μm). Data was acquired in the q-range from 0.01 to 0.95 $Å^{-1}$ with a measurement time of 28800 s for each scattering pattern at vacuum conditions better than $10^{-2}$ mbar. In-situ heating experiments were carried out with the aid of a Linkam HFSX 350 heating stage. Subsequent data manipulation included background correction based on the measured transmission and radial integration with the SaxsGui 2.8.03 software package. In cases of large deviation in the capillary dimensions (+/- 5%) the transmission was normalized with the absorption of water to an equivalent nominal thickness. Therefore the outer diameter of the capillaries was measured using a light microscope (Leica DM 4000 M). The thickness of the walls was equal and therefore needed no correction.

*10.2 SAXS data fitting model*

A theoretical model of the shell morphology was developed based on a mean field statistical approach established by Daoud & Cotton for polymer brushes. According to the Daoud - Cotton model, the brush can be represented by a sequence of concentric close packed blobs. Within every blob, chains behave as if it would be free, hence following the free chain scaling laws. Based on this theoretical approach, the local monomer concentration can be computed and estimated as a function of the distance r from the grafting point. The shell of each coated magnetite core can be described as a star polymer with f branches, each one consisting of N statistical units of lengths l, where 1 is taken to be half of the Kuhn length. Due to the spherical symmetry of the system, the polymer brush covering the colloid is represented as a sequence of concentric blobs, of radius ξ(r). Each blob contains a certain number of statistical unit lengths, which within every blob behaves as an insulated polymer. Given the spherical symmetry, blobs are contained in cones; the closer to the surface the smaller the radius of the blob. Since blobs are close packed, by purely geometrical consideration the radius ξ(r) of the blob scales with the number of branches f and the distance from the grafting point r as: $\xi(r) \sim r f^{-\frac{1}{2}}$ The closer the blobs are to the grafting surface, the smaller their radius. It is possible to distinguish three distinct statistical

regions. The first region $r<r_1=l^*f^{1/2}$ is characterized by a constant density of monomers. In this region, the grafted chains are so closely packed that the monomers behave as if they were in a melt. Originally called core region, the term melt region or melt like region is used to avoid confusion with the iron oxide cores used as the grafting surface.

The regimen $r_1<r<r_2$ is termed the "unswollen" region. Here the radius $\xi(r)$ of a blob is smaller than that of a thermal blob and the polymer cannot behave as an ideal, self-avoiding chain but are affected by the solvent quality that find its expression in the excluded volume parameter via the Flory interaction parameter.

The second demarcation distance $r_2$ is given by $r_2=l^*\bar{v} * f^{1/2}$ with $\bar{v}=v/l^3$ being the dimensionless excluded volume parameter. For distances $r > r_2$, the radius of the blob $\xi(r)$ is bigger than that of a thermal blob. The $r>r_2$ region is the so called "swollen" region; here the blobs follow a self-avoiding polymer statistics and their radius scales with the number of monomers in it to the power 3/5:

$$\xi(r) \sim n(r)^{\frac{3}{5}}.$$

For the whole polymer shell with the distinct regimens the concentration of monomers within the blobs is given by:

$$c(r)\begin{cases} = const, & for\ r < r1 \\ \sim \dfrac{n(r)l^3}{\xi^3(r)}, & \text{for } r_1 < r < r_2 \\ \sim f^{\frac{2}{3}}(\frac{r}{l})^{-\frac{4}{3}}a^{-3}\bar{v}^{-\frac{1}{3}}, & \text{for } r > r_2 \end{cases}$$

The excluded volume parameter $\bar{v}$ for PEG in good solvent is one, therefore in good solvent $r_1=r_2$ and hence the intermediate regimen vanishes. For $r>r_2$ the number n(r) of statistical segments within a blob of radius $\overline{\xi(r)}$ is smaller than those in the thermal blob and within each blob the chains behave as if they were ideal chains. In this region, the concentration follows the usual star polymer $r^{-4/3}$ decay. A graphical representation of those distinct regimens is given in Fig. 9.

This behaviour can easily be transferred into an electron density profile as shown in Fig. 10. Note that the scattering length density (electron density) of the shell is not representative of that in the real core-shell particles used here but greatly exaggerated for reasons of better visibility.

As the x-ray scattering is given by the Fourier transformation of the scattering length density variations encountered in a system, the form factor F(q) of a core-shell particle can be obtained

$$F(q) = 4\pi\int_0^\infty SLD(r)\frac{\sin(qr)}{qr}dr, q$$

With SLD(*r*) being the scattering length density profile of the particle and q being the scattering vector can be calculated for a wavelength $\lambda$ of the incident x-rays and an angle $\theta$ of the scattered waves as:

$$q = \frac{4\pi\sin(\theta)}{\lambda}$$

The scattering length density in SAXS is intimately related to the monomer density c by the coherent scattering length $b_{coh}$

$$SLD = c * b_{coh}$$

The coherent scattering length $b_{coh}$ is proportional to the cumulative atomic number Z of the monomer and the classical electron radius.

$$b_{coh} = Z * r_e$$

If core size and the polymer grafting density are known, one can directly calculate the monomer concentration, the scattering length density as well as the scattering length density contrast and compare the numbers to the SAXS data. The monomer density in the shell is calculated as follows:

As a first step, we obtain f, the number of PEG molecules attached to the core surface calculated grafting density $\rho_{graft}$ and the surface of the core to obtain

$$f = \rho_{graft} * 4 * R_c^2 * \pi \quad \text{(eq. 1)}$$

A monomer concentration c(r) varies with the radial distance to the center of the particle. The total number of monomers in the shell, $n_{mono}$ is given by the integral of c(r) over the shell thickness and equals the product of the number of polymer chains f and the chain length N:

$$n_{mono} = 4\pi \int_{R_c}^{R_p} c(r) * r^2 * dr = N * f$$

In the case of a density decay with $r^{-4/3}$ as predicted for star polymers, it follows that:

$$\mathrm{N} * \mathrm{f} = \int_{R_c}^{R_p} c(R_c) * \left(\frac{r}{R_c}\right)^{-4/3} * r^2 * dr$$

By integration and solution of the equation the monomer concentration near the core c(Rc) can be expressed as follows

$$c(R_c) = \frac{3}{4\pi} \frac{5 * N * f}{9 * R_c^{4/3} (R_p^{5/3} - R_c^{5/3})}$$

In case of a high grafting density (large number of chains f), the Daoud-Cotton model predicts a constant density region up to a radius r<r1=l*f1/2 $c(R_c)= c(r_1)$ as shown in Fig.10 which is added to the star polymer decay. To be of practical relevance, the melt-density regimen has to exceed the core radius. The necessary number of chains f attached to the core can be calculated as:

$$f = \left(\frac{\bar{\nu} * r1}{a}\right)^2$$

The monomer concentration between the core and r1=r2 is then given by

$$\mathrm{c}(\mathrm{r}_1) = \frac{3}{4\pi} \frac{5 * N * f}{5(r_1^3 - R_c^3) + 9 * r_1^{4/3} * (R_p^{5/3} - r_1^{5/3})}$$

Thereafter it decays with $r^{-4/3}$ as for star polymers.

As mentioned above, the monomer density can be directly used to calculate the scattering length density. In practice, it has to be considered that the SAXS signal will depend on the scattering length density contrast of core and shell with respect to the solvent. Therefore a relative scattering length density of the shell compared to that of the core (always with respect to the solvent water) is defined. It is a measure of how much the shell contributes to the SAXS signal and therefore allows an estimate on the visibility of the shell

$$\Delta SLD = \frac{SLD_{Shell} - SLD_{H2O}}{SLD_{Fe3O4} - SLD_{H2O}}$$

The model was implemented in a Mathematica 9 script and fitted to the data in a q range from 0.045 to 0.49 $Å^{-1}$, using the free variables of particle diameter, ΔSLD and a scaling parameter. The value for the core radius was fixed to the values determined separately by the TEM measurements. The value for the constant density radius was calculated based on the morphology of the shell. Fitting was carried out using a Levenberg-Marquardt algorithm minimizing fit residuals and evaluated by means of the coefficient of determination R2 for the whole fit and the estimated standard errors for each fit parameter.

In order to carry out the quantitative modelling of the core-shell size, the SLD parameters haven been determined based on the set of equations a ΔSLD of 0.025 is calculated as the starting value of the fit parameters together with an estimate of 8 nm for the particle radius $R_P$.

It has to be pointed out that the model does not take account any effects of instrumental resolution and polydispersity. Albeit being low, a certain "smearing" of the data is inevitable. As no dedicated variable could be defined for this, the ΔSLD, which is also affected by polydispersity and instrumental resolution, was effectively used as a fit parameter in the current model.

Fig. 11 shows the scattering from the dry cores (red squares) with the fit-function (black line) for a sphere, with gaussian size distribution and the structure factor for hard spheres (black line). The diameter was determined to be 4.6 nm with a polydispersity of 17 %. The same function was also applied to q> 0.14 $A^{-1}$ region of the core-shell particles in order to fit the size and polydispersity of the core signal alone.

**Table 2 Core diameter (D) and polydispersity (SD) as established by SAXS and TEM**

| Sample | D SAXS [nm]/SD | TEM D Pebbles [nm]/SD[nm]/N | TEM D Manual [nm]/SD[nm]/N |
|---|---|---|---|
| Bare Fe3O4 cores | 4.6± 0.34 | 4.6 ± 0.30<br>1360 | 4.35 ± 0.25<br>252 |
| Core-Shell particles, core | 3.7± 0.42 | 3.8 ± 0.4<br>1209 | 3.4 ± 0.3<br>200 |

**Table** 2 gives a comparison of the different methods of size determination and their respective results. It can be seen that all methods are in good agreement with each other to within experimental uncertainty. The Pebbles evaluated TEM diameters are in good agreement with the diameter determined by SAXS. The manual analysis of the cores in the TEM images yields systematically smaller diameters which may owe to the fuzzy interface area between cores and the grey scale fitting algorithm of Pebbles that utilizes the sphericity of the nanoparticles to obtain a more precise size fit to the image data.

Nevertheless, a good agreement between TEM and SAXS methods, in particular with Pebbles fitted data, can be seen which justifies the direct application of TEM values for the modelling of the core-shell particle scattering.

TGA, grafting density, calculation of SLD.

From eq. 1 the monomer density in the dense melt like region at the core can be found to be $1.18*10^{22}$ / $cm^3$, which seems reasonable in comparison to 1.55 $*10^{22}$ / $cm^3$ as calculated for a PEG melt.

The scattering density of the PEG shell at the core follows from this as $7.85*10^{10}$ $cm^{-2}$. With a scattering length density of $4.11*10^{11}$ $cm^{-2}$ for Magnetite and $9.43*10^{10}$ $cm^{-2}$ for $H_2O$, ΔSLD for the model follows as 0.025 in comparison to 0.033 for the pure melt.

*10.3 Shell*

The thickness of the PEG polymer shells grafted to nanoparticles is highly dependent on the solvent, which is crucial to their functionality in biological environments. Measurements were carried out in MQ water and buffer solutions. Figure 12 shows a typical scattering curve from a core-shell particle in water and exhibits two distinct features. One in the larger q range from 0.23 to 0.42 $A^{-1}$, which can be attributed to the core signal as in Figure 11 and a shoulder with a weak peak in the region of 0.1 $A^{-1}$, where 0.1 $A^{-1}$ in the reciprocal space corresponds to 6.3 nm in real space.

A dilution series was carried out to check on possible particle-particle correlations.

The scattering curves in Figure 12 depict the scattering from particles with decreasing concentration. The shoulder around 0.12 $A^{-1}$ keeps its position and the whole signal decreases with decreasing concentration. It is therefore concluded that the peak at 0.12 $A^{-1}$ cannot be attributed to a concentration dependent structure factor but that it is due to permanent structural features such as the shell. It should be noted that a structural peak at 0.12 $A^{-1}$ would correspond

to an interparticle distance of 5.2 nm and could therefore only be caused by corecore interaction. Therefore the absence of a concentration dependent structure peak in the q-range corresponding to the core diameter is a good indicator for the integrity of the PEG shell effectively preventing core agglomeration.

*10.4. Size and scattering contrast of the PEG shell*

Having ruled out a concentration dependent structure peak, we attribute the shoulder around 0.12 $A^{-1}$ to the PEG shell. The scattering curve can thus be used to fit the size of the shell. Figure 13 displays the scattering data of the core-shell particles in MQ and the respective fit with obtained $R_p$ of 8.2 nm. The fit is consistent with a constant density of the core, a highdensity region of PEG close to the core and a decrease of PEG density according to $r^{-4/3}$ in the outer part of the shell as predicted for star polymers in a good solvent. The monomer density at the core is found to be 1.18*$10^{22}$ $cm^{-3}$ which compares well to the 1.55 *$10^{22}$ $cm^{-2}$ in the PEG melt.

The actual curve fitting gave a value for the SLD of 0.015 (Table 3). This is lower than the theoretical estimate 0f 0.025 calculated for the particles and therefore reasonable but still reasonable as it is not only influenced by the actual conformation of the brush but as well by experimental factors or assumptions of the underlying models such as homogenous hydration of the shell or the monodispersity of the shell.

Furthermore, this consistent curve fitting yielded a value for $r_2$ of ~4.6nm. The use of a traditional spherical brush Gaussian or parabolic density profile did not produce a good fit to the data. The fitted value of $r_2$=4.6nm means a constant density, melt like region, with thickness of more than 2, almost 3 nm for the small cores used in the experiment. For larger nanoparticles with lower curvature, the thickness of this region would increase at the same grafting density.

Generally speaking, the core-shell particles exhibit a remarkable monodispersity both for core and shell features which justifies the approach used for modelling here. Substantial polydispersity would smear out the features to an extent not observed in the investigated nanoparticle system; indeed, it would prohibit the performed fit analysis.

*10.5. Cloud point buffer experiments*

In order to test the hypothesis of dumbbell formation as an origin for the shoulder/peak at 0.12 $A^{-1}$, the swelling behavior of the PEG shell in cloud-point buffers was monitored by SAXS measurement of PEG coated particles in cloud-point buffers. When polymer coated nanoparticles are dispersed in buffer solutions, temperatures close to the cloud point of the buffer should result in a collapse of the shell visible in the SAXS curve, whereas dumbbells would not be affected by temperature changes. A cyclic temperature program was chosen with an initial measurement at 25°C, one point at elevated temperature in the vicinity of the cloud point (50°C) and a final cooling step back to the original conditions (25°C). Free PEG polymer is known to demonstrate clouding under these buffer conditions in the temperature range 70-80°C due to specific ion interactions, which can decrease for a high volume fraction of PEG. As the temperature is increased towards this "cloud point" the polymer excluded volume is expected to decrease; in our case the shell size should decrease. This transition is broader than a LCST transition in pure solvent.

The results show that the peak/shoulder is indeed responsive to changing temperature, as shown in Figure 14. It is therefore concluded that the shoulder can be attributed to the PEG shell. The shell in its initial stage at 25°C (first step, 25°C, green) has size of 8.8 nm, comparable to the size observed in MQ. Heating to 50°C (second step, red) brings the shell to its cloud-point which shrinks the shell radius to 7.5 nm. The final cooling step (third step, cyan) expands the shell to 8.6 nm, which is in close proximity to the initial shell radius. The observed changes in shell size upon temperature are significant. A temperature induced reduction in hydration is therefore observed and a structural origin of the peak/shoulder can be further ruled out.

In Figure 15, the simulation of intensity-normalized scattering curves for the different shell sizes is shown in the q-region of the shell (0.01 to 0.1 $A^{-1}$) as well as an inset with the scattering curve of the whole core-shell particle. Observable is the shift of the shoulder in the 50°C step towards larger q.

**Table 3.** Parameters for shell diameter, ΔSLD and R2 as obtained by the curve fitting with the respective error estimates.

| Sample | $D_{Shell}$ | SLD |
|---|---|---|
| Core-Shell particles in MQ | 16.4±0.2 | 0.0143±0.0007 |
| Cloudpoint 25°C First Step | 17.7±0.2 | 0.0119±0.00028 |
| Cloudpoint 50°C Second Step | 14.9±0.2 | 0.0248±0.0001 |
| Cloudpoint 25°C Third Step | 17.2±0.2 | 0.0195±0.0004 |

Table 3 shows the obtained fit parameters for the shell diameter and the SLD. $R^2$ for the fit was in all cases better than

0.99. As discussed in the "Shell" section the results are consistent as they indicate a monomer density lower than the upper theoretical limit given by the Daoud-Cotton model. In the case of the cloud point temperature cycle the values show a large variability for the different temperatures. That indicates that the quite straightforward system that is observable in MQ gets more complicated due to necessary interactions between the buffer, its ions and the PEG polymers. The SLD parameter only governs the definition and shape of the shoulder and not its position; thus, the observed change in the shoulder position is attributed to shrinkage and subsequent swelling of the shell during a change of temperature. This experiment shows not only the reversibility of the cloud-point behavior of the particles but also shows that the shell always maintains a clearly defined size with low polydispersity as otherwise the signal would vanish.
This observation basically indicates three points:

- Shoulder stays in the same region meaning a comparatively low response to change in environment
- Increasing temperature causes a decrease in the shoulder intensity and shift of the shoulder to larger q (smaller radius) meaning no dumbbells (structure contribution)
- The change of the q position is reversible
- The shoulder can hence be attributed to the PEG shell which shows reversible shell shrinkage in the vicinity of the cloud point.
- A constant density region relating to a molten like state of this region of the shell was observed close to the iron particle, with a thickness of about 2.75 nm.

These observations have not been shown for particles grafted by the direct method used by Amstad et al. 2009, supra. In fact, for grafting densities below 1 M/nm$^2$, a shell could not be observed by SAXS using the same experimental setup since the $\Delta$SLD was too low.

**Example 11: Comparison of surface density estimation methods**

The high dispersant density of the inventive particles has not been observed on truly monodisperse and spherical nanoparticles, and higher numbers might partly be a consequence of higher surface roughness and effective surface area. Thus, the effective grafting density is likely below 1.0 M/nm$^2$ also for these nanoparticles used in our early work (Amstad et al. 2009, supra.).

Comparison of the absolute dispersant density per nm$^2$ requires that the same measurement and calculation methods are applied to all samples. The samples should be fully comparable. It further requires that care has been taken to remove all free dispersants from solution before quantification. Therefore, stringent purification by removal of free dispersant, e.g. through a Sephadex (dextran) column that shows high affinity to iron oxide nanoparticles, is a good way of demonstrating high colloidal stability and high dispersant density. However, the differences in purification method, characterization method and calculation method, makes numbers from the literature difficult to compare, which is well established in the field.

Nanoparticles have a very high surface-to-volume ratio. For spherical nanoparticles, a difference in a few Å (corresponding to single atomic layers) in terms of determining the particle radius can have dramatic impact on the calculated dispersant density determination, since the area used for the calculation scales with the square of the radius. Similarly, calculating the area from the radius measured by TEM, SAXS or other common methods assuming a sphere, can strongly underestimate the actual area of a non-spherical nanoparticle, since the minimum area for a 3D object is obtained for a sphere. The dispersant grafting density is calculated from the number of dispersants measured by a chosen characterization method, usually TGA which requires no assumptions on the physical properties of the organic molecules, normalized to the surface area; thus, underestimating the surface area will artificially increase the estimated grafting density. TGA, however depends on the choice of thermal interval investigated, the atmosphere under which the thermal decomposition of organics take place, and for high organic fraction materials (densely polymer grafted nanoparticles) can have a relatively large error. Other methods making use of spectroscopy, however, have to rely on the correct assignment and integration of peaks, correct calibration to a known peak, the correct attribution of relative cross-sections or extinction coefficients. All these uncertainties are well-known to someone experienced in the field. Therefore, relative comparisons for different grafting methods on the same particles under the same conditions is the preferred way to establish superior methods rather than comparing absolute values from different types of methods and preparations throughout the nanoparticle literature. For the inventive two-step grafting-to method using a fluidized dispersant, this comparison was performed in relation to the previous standard direct grafting-to method.

Previously, a grafting density as high as 2.8 M/nm$^2$ has been reported using the same type of binding chemistry but using the direct grafting-to method and polydisperse iron oxide cores formed by thermal decomposition (Amstad et al., 2009, supra). Calculation of the grafting density for these samples relied on the above assumptions, which as illustrated in Fig. 16 will result in a significant overestimation of the grafting density, when polydispersity and non-sphericity is taken into account, i.e. the effective surface area for grafting will be strongly underestimated.

Comparing the direct grafting-to method to the two-step method using a fluidized dispersant on monodisperse spherical nanoparticles elucidates the much higher grafting density achieved by the latter method compared to the first. This is a direct relative comparison of the same nanoparticle cores after grafting and removal of excess dispersants using the same TGA settings and calculations for characterization. The results reported in Table 1 demonstrate an at least 2-3 times higher grafting density of the latter compared to the first, which are shown to translate also into a unique structure of the shell with near maximum density of the polymer close to the inorganic core as theoretically predicted.

This difference could further be studied by SAXS, as described in Example 10, which could detect the shell around densely grafted particles and match the density profile to the theoretical predictions; while the direct grafting-to method did not lead to polymer shell densities observable by SAXS (the scattering length density contrast is too small for strongly hydrated PEG). The constant high density, melt like, region of the dispersant shell was shown to extend several nm from the inorganic core surface. This provides the core-shell nanoparticle with the surface and interaction properties of that of a star polymer (cf. Daoud Cotton) comprising the dispersant polymer, completely masking inorganic core interactions. The physical properties of such core-shell nanoparticles are thus qualitatively different to those of more sparsely grafted core-shell nanoparticles.

## Claims

1. A method of producing inorganic core particles comprising a dispersant shell, comprising a dispersant molecule in a high surface covering density on the inorganic core, comprising the steps of:

   - providing one or more inorganic particles,
   - ligating at least one organic linker onto the inorganic particle, thereby obtaining an inorganic core linker coated particle,
   - providing at least one fluidized dispersant, preferably in form of a melt, suspension or solution,
   - binding the at least one fluidized dispersant to the at least one organic linker, thereby obtaining the inorganic core particles comprising a dispersant shell.

2. The method of claim 1, comprising raising the temperature of the dispersant above its melting temperature and performing binding above the melting temperature.

3. The method of claim 1 or 2, wherein the dispersant is a macromolecule, preferably a macromolecule comprising a polymer, preferably PEG, polyoxazoline, poly(N-isopropylacrylamide), polyisobutylene, caprolactone, polyimide, polythiophene, polypropylene, polyethylene, polyacrylic acids and other polyelectrolytes, polyvinylpyrrolidone, polyvinylalcohol.

4. The method of any one of claims 1 to 3, wherein the dispersant has an average weight of 1 kDa to 30 kDa, preferably of 2 kDa to 15 kDa, especially preferred of 3 kDa to 10 kDa.

5. The method of any one of claims 1 to 4, wherein the inorganic core is paramagnetic, preferably superparamagnetic.

6. The method of any one of claims 1 to 5, wherein the inorganic core comprises a metal, preferably selected from Fe, Ti, Ni, Co, or an oxide any thereof, preferably a Fe oxide, such as $Fe_2O_3$ and/or $Fe_3O_4$, or comprises a metalloid or a semiconductor or consists of a non-metal material, preferably the inorganic core comprises Al, Si, Ge, or silica compounds.

7. The method of any one of claims 1 to 6, wherein the inorganic core has an average diameter size of 1 nm to 400 nm, preferably 1.5 nm to 100 nm, especially preferred 1.8 nm to 12 nm, 2 nm to 20 nm, 3 nm to 40 nm, 4 nm to 80 nm.

8. The method of any one of claims 1 to 7, wherein the linker and the dispersant each comprises one member of a pair of reactive groups, capable of forming a chemical bond, or further members of further such pairs,
   preferably wherein the pair of reactive groups is selected from an amine and an aldehyde, an amine and an acrylate, an amine and a carboxyl group, an amine and an ester group, a sulfonylchloride and either an amine or a hydroxy-group, an acid-chloride and either an amine or a hydroxy-group, a thiol and any one selected from a maleimide, an acrylate or an allyl-group, an isocyanate and either a hydroxy-group or an amine, a conjugated dien and a substituated alkene, an epoxide and a nucleophile,
   preferably wherein an amine and an aldehyde is reacted n the binding reaction, thereby forming an imine, which is even more preferred reduced to an amine by a reducing agent, such as $NaCNBH_3$.

**9.** The method of any one of claims 1 to 8, wherein said linker or dispersant comprises a leaving group that is removed in the ligation reaction and/or wherein binding the linker to the dispersant is a reaction without the requirement of a solvent.

**10.** The method of any one of claims 1 to 9, wherein the linker comprises an alcohol or carboxyl group in contact with the surface of the inorganic core, preferably a moiety selected from a 6 membered homocycle comprising oxygen or hydroxyl substituents, such as benzoic acid, phenol or catechol, and/or preferably wherein the linker molecules comprise an aromatic group with a electronegative group bound thereto, more preferred a nitro group, especially preferred wherein the linker molecules comprise a moiety selected from nitrocatechol, nitroDOPA or nitro-dopamine.

**11.** The method of any one of claims 1 to 10, wherein the concentration of the dispersant is above the solubility concentration of a solvent thereby obtaining a suspension and wherein the dispersant is fluidized by increasing the temperature above the melting temperature of the suspension.

**12.** The method of any one of claims 1 to 11, wherein the provided inorganic particle is in complex with a surfactant on the particle surface, especially preferred in case of metal particles, and wherein ligating the organic linker onto the inorganic particle is by replacing the surfactant by said organic linker, thereby obtaining an inorganic core linker coated particle.

**13.** The method of any one of claims 1 to 12, wherein the step of providing an inorganic particle comprises providing a plurality of inorganic particles wherein the mean standard deviation of the particle's average size is at most 10%, preferably at most 5%, even more preferred at most 2% of the particle's average size, such as at most 0.8 nm, preferably at most 0.5 nm.

**14.** A particle comprising an inorganic core surrounded by linkers that are chemically linked to dispersant molecules, wherein the dispersant molecules

(a) are at an average density of at least 1.1, preferably at least 3.0, dispersant molecules per $nm^2$ of the inorganic core surface, and/or
(b) form a shell of constant dispersant density and a further shell of gradually reduced dispersant density with increasing distance from the inorganic core surface, preferably said particle being obtainable by a method of any one of claims 1 to 13.

**15.** A preparation of a plurality of particles, wherein

- said particles comprise an inorganic core surrounded by linkers that are chemically linked to dispersant molecules,
- wherein the dispersant molecules

(a) are at an average density of at least 1.1 dispersant molecules per $nm^2$ of the inorganic core surface, and/or
(b) form a shell of constant dispersant density and a further shell of gradually reduced dispersant density with increasing distance from the inorganic core surface, and

preferably wherein the inorganic core is of an average size between 2 nm to 80 nm in diameter and further of homogenic size in said plurality wherein the mean standard deviation of said average size is at most 10%, preferably at most 5%, even more preferred at most 2% of said average size, such as at most 0.8 nm, preferably at most 0.5 nm, preferably said preparation being obtainable by a method of any one of claims 1 to 13.

Fig. 1

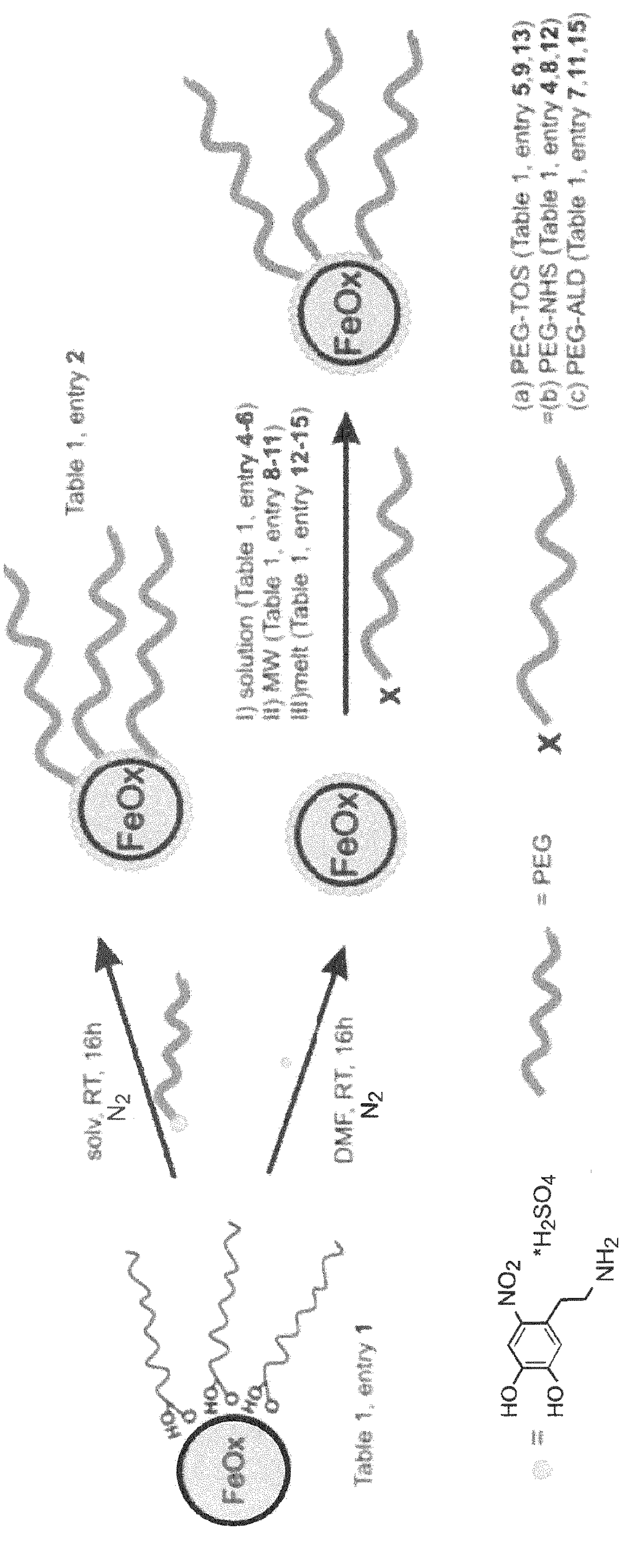
FeOx
Table 1, entry 1
solv, RT, 16h
$N_2$
DMF, RT, 16h
$N_2$
Table 1, entry 2
i) solution (Table 1, entry 4-6)
ii) MW (Table 1, entry 8-11)
iii) melt (Table 1, entry 12-15)
X
= PEG
(a) PEG-TOS (Table 1, entry 5,9,13)
=(b) PEG-NHS (Table 1, entry 4,8,12)
(c) PEG-ALD (Table 1, entry 7,11,15)

Fig. 2

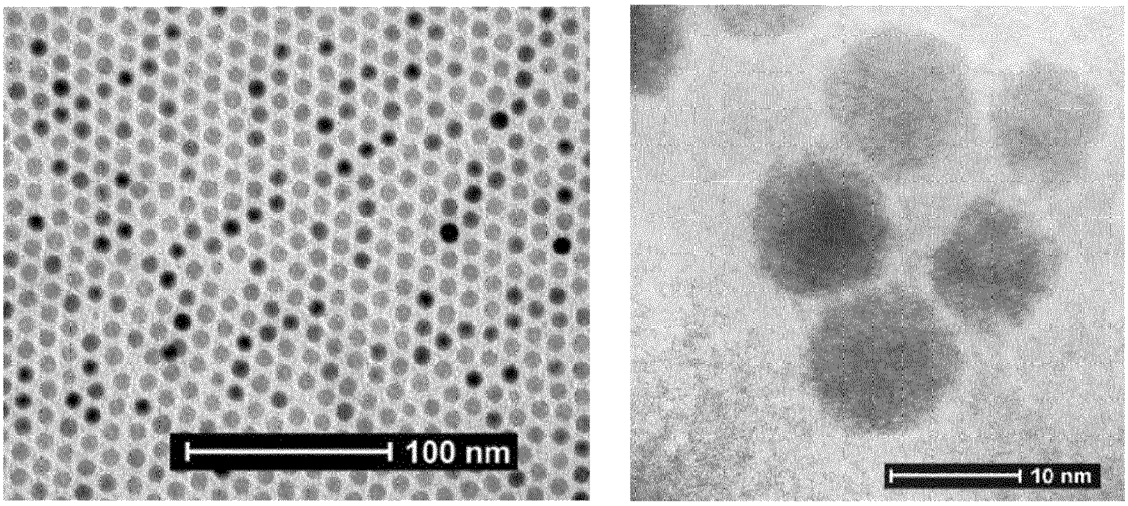
100 nm
10 nm

Fig. 3

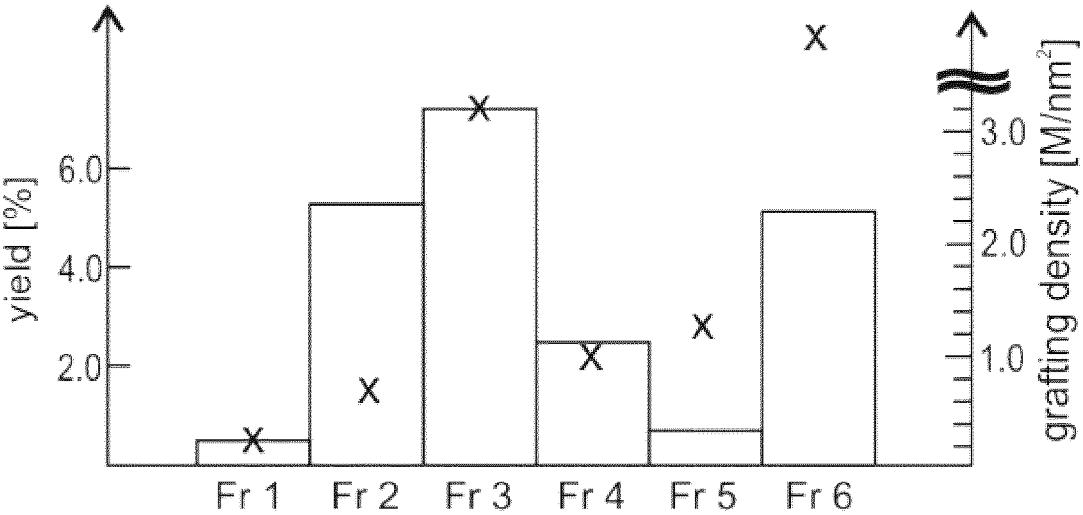
yield [%]
6.0
4.0
2.0
grafting density [M/nm$^2$]
3.0
2.0
1.0
Fr 1
Fr 2
Fr 3
Fr 4
Fr 5
Fr 6

Fig. 4

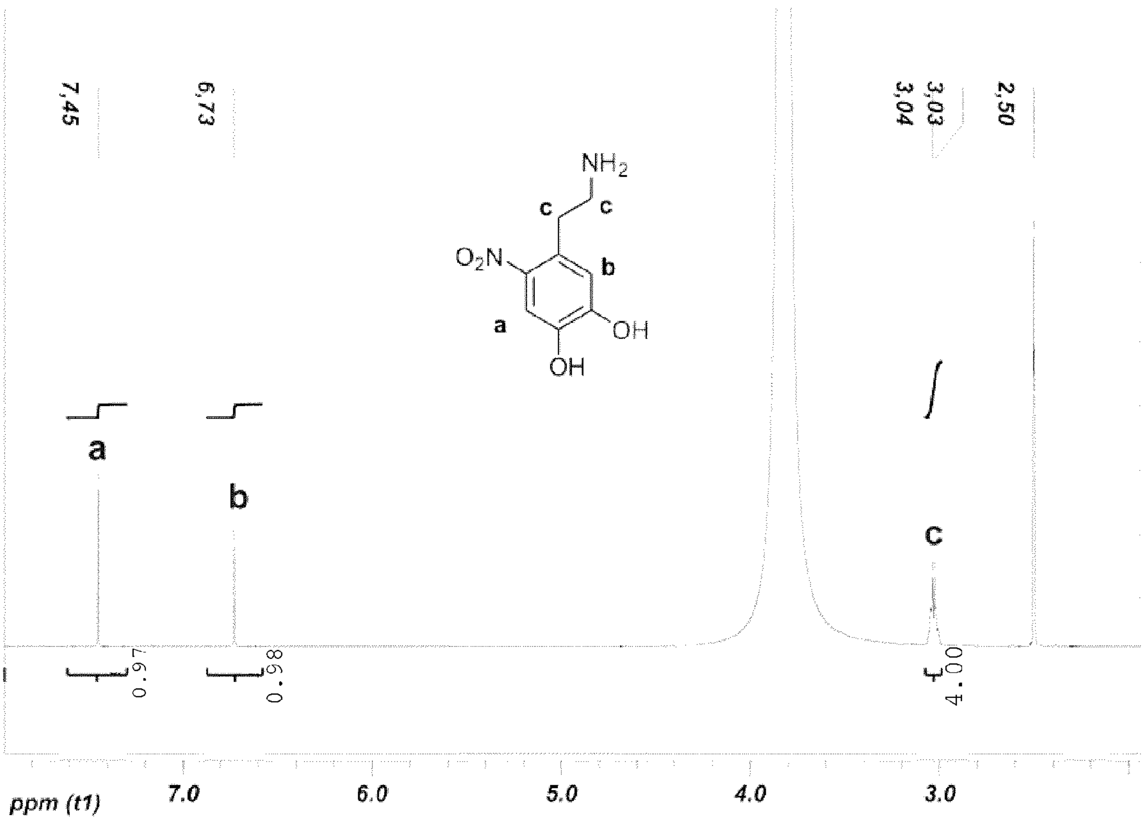


Fig. 5

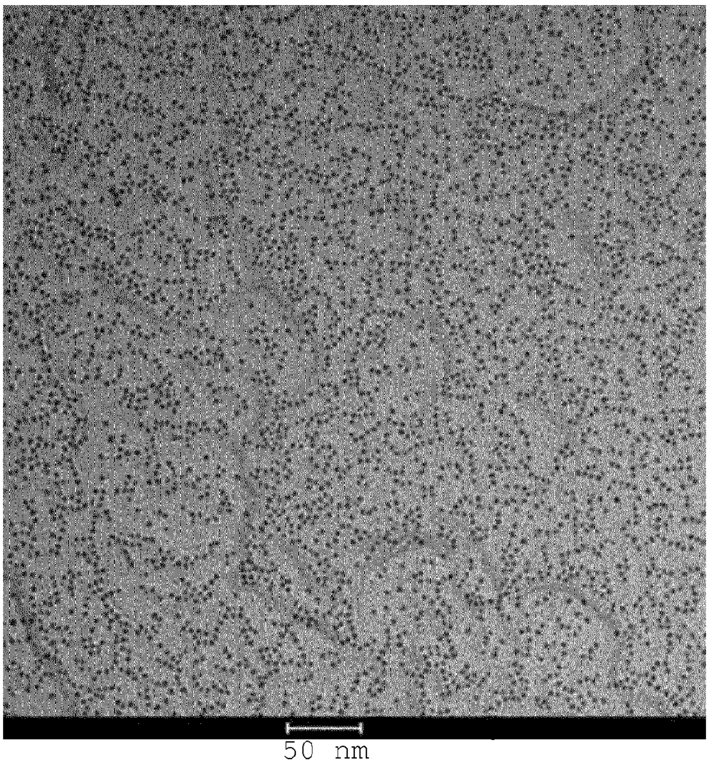

Fig. 6

a)

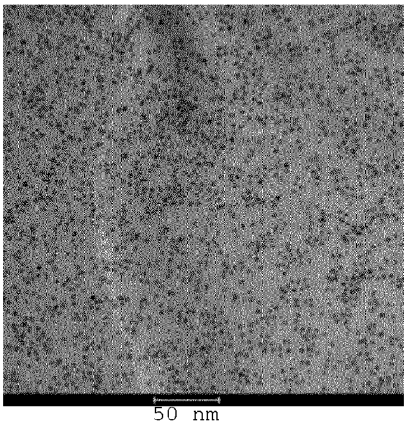
50 nm

b)

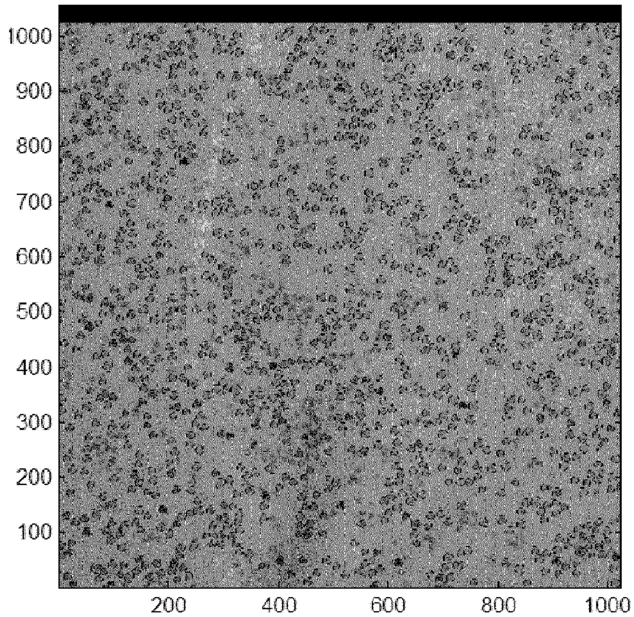
1000
900
800
700
600
500
400
300
200
100
200
400
600
800
1000

c)

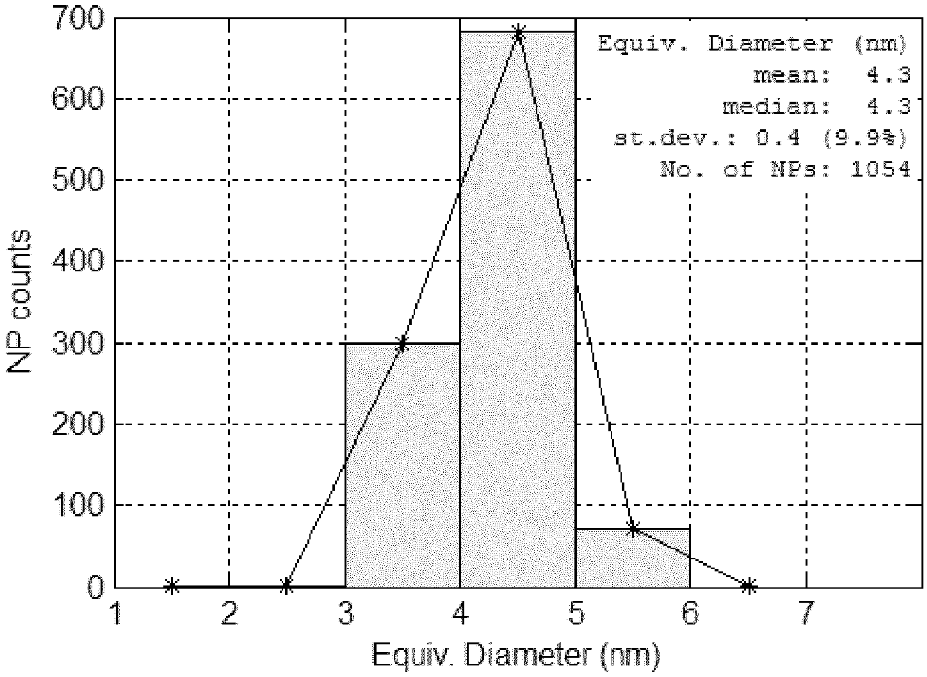
Equiv. Diameter (nm)
mean: 4.3
median: 4.3
st.dev.: 0.4 (9.9%)
No. of NPs: 1054
NP counts
Equiv. Diameter (nm)
0
100
200
300
400
500
600
700
1
2
3
4
5
6
7

Fig.7

a)

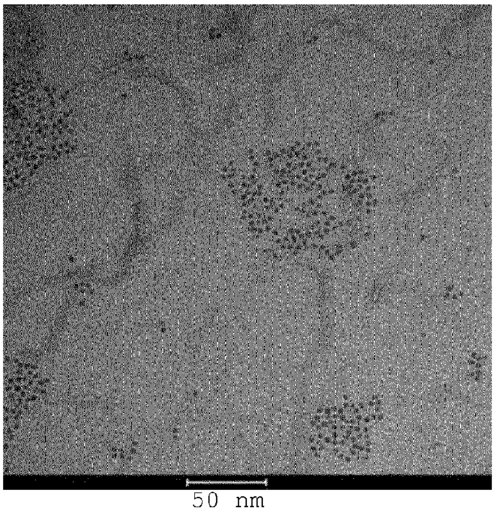


b)

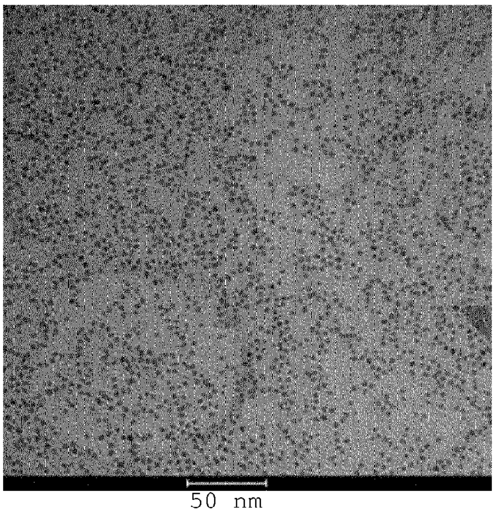


c)

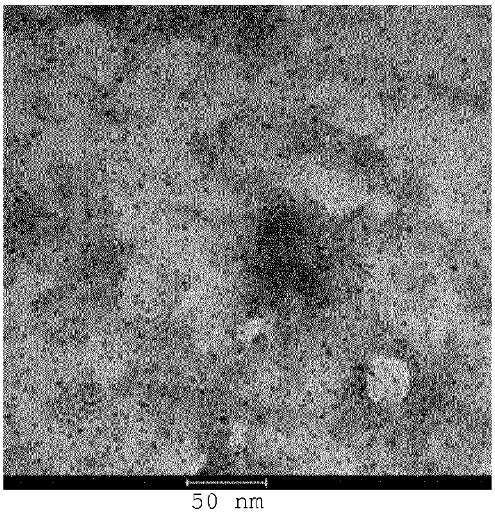

Fig. 8

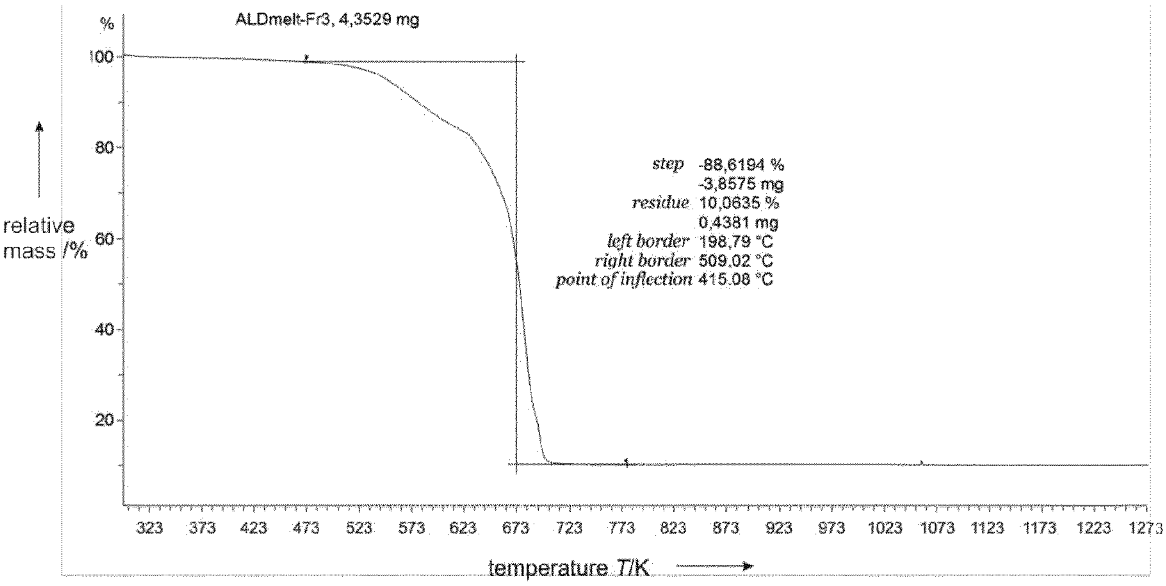
%
ALDmelt-Fr3, 4,3529 mg
100
80
60
40
20
relative mass /%
step -88,6194 %
-3,8575 mg
residue 10,0635 %
0,4381 mg
left border 198,79 °C
right border 509,02 °C
point of inflection 415.08 °C
323 373 423 473 523 573 623 673 723 773 823 873 923 973 1023 1073 1123 1173 1223 1273
temperature T/K

Fig. 9

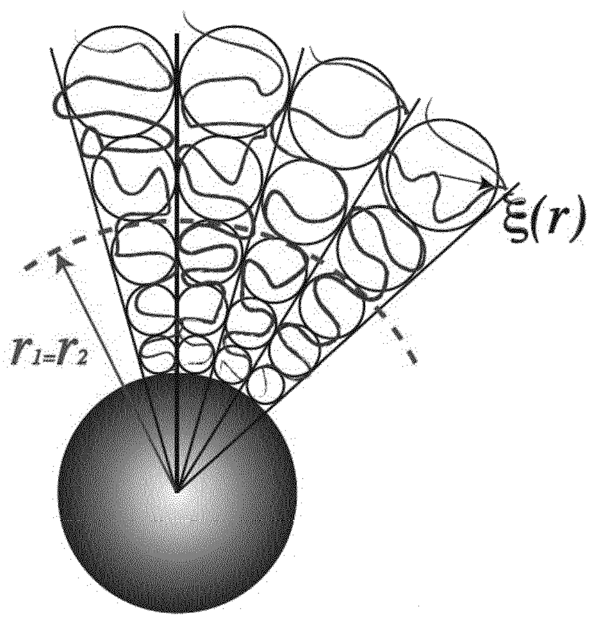
ξ(r)
r1=r2

Fig. 10

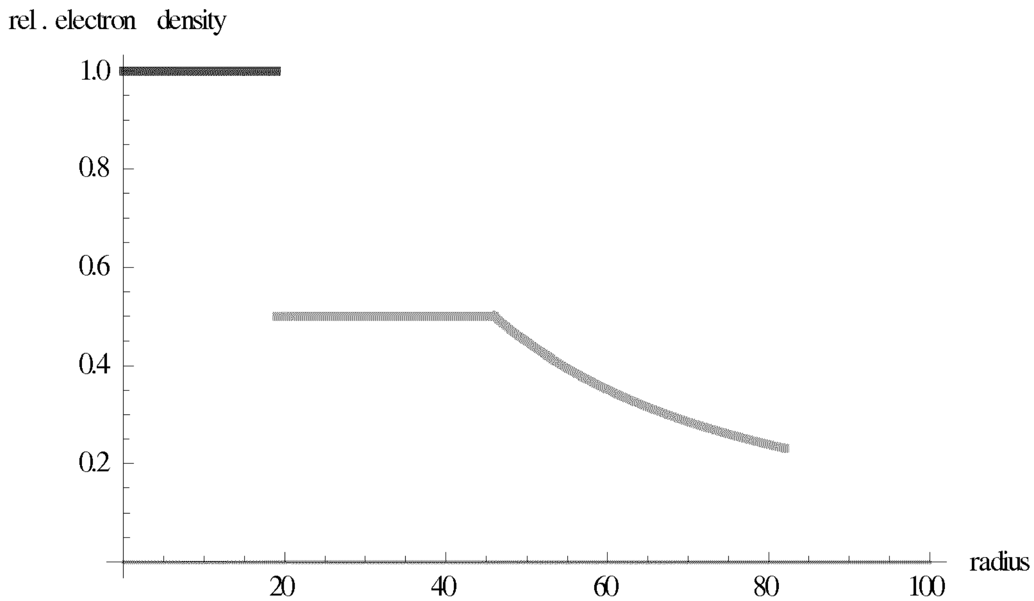
rel. electron density
1.0
0.8
0.6
0.4
0.2
20
40
60
80
100
radius

Fig. 11

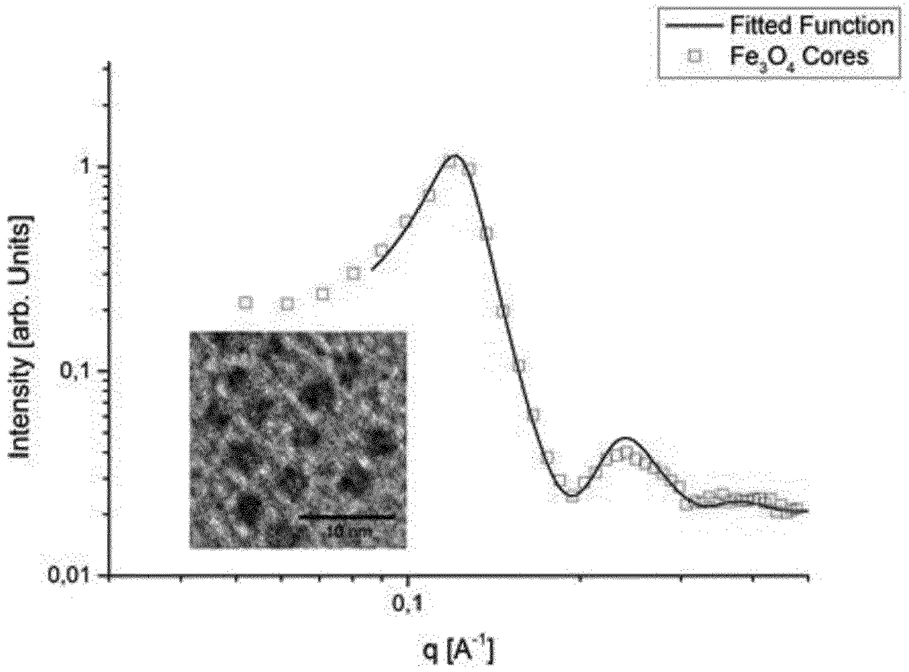
Fitted Function
$Fe_3O_4$ Cores
Intensity [arb. Units]
1
0,1
0,01
0,1
q [A$^{-1}$]
10 nm

Fig. 12

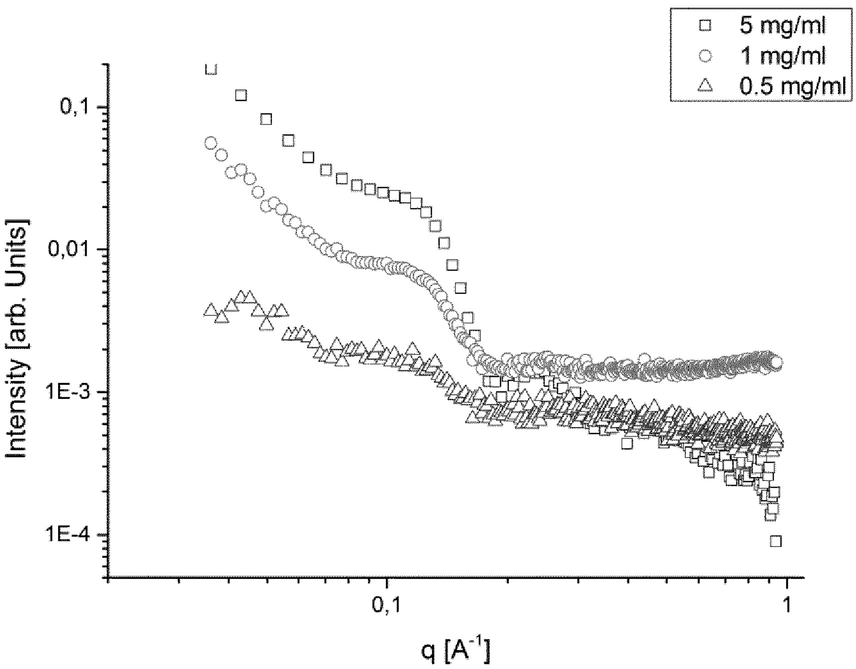
5 mg/ml
1 mg/ml
0.5 mg/ml
Intensity [arb. Units]
0,1
0,01
1E-3
1E-4
0,1
1
q [A$^{-1}$]

Fig. 13

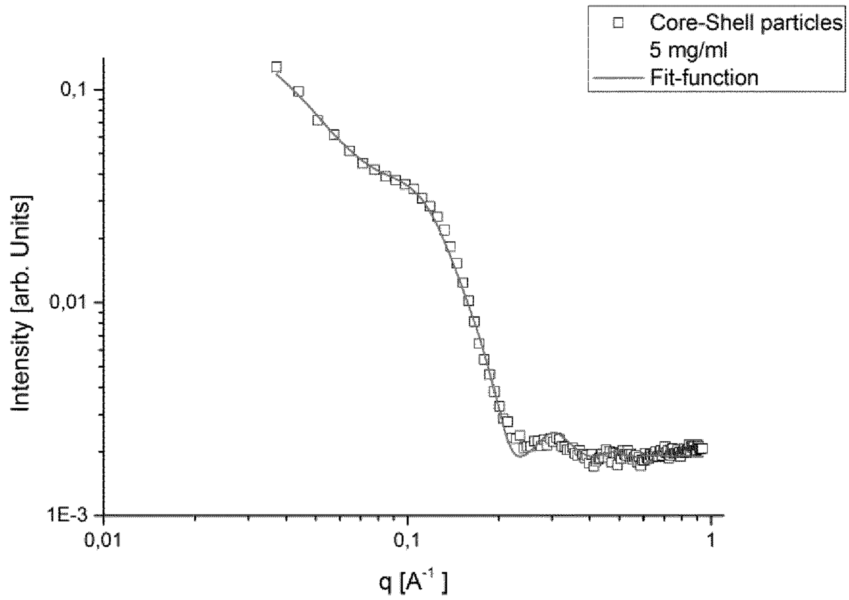
Core-Shell particles
5 mg/ml
Fit-function
Intensity [arb. Units]
0,1
0,01
1E-3
0,01
0,1
1
q [A$^{-1}$ ]

Fig. 14

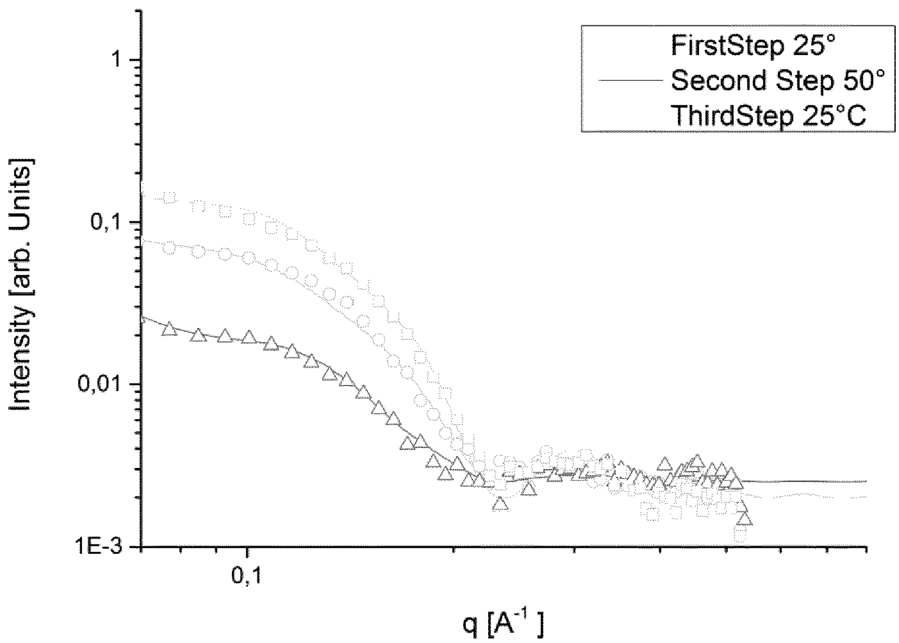
FirstStep 25°
Second Step 50°
ThirdStep 25°C
Intensity [arb. Units]
1
0,1
0,01
1E-3
0,1
q [A$^{-1}$ ]

Fig. 15

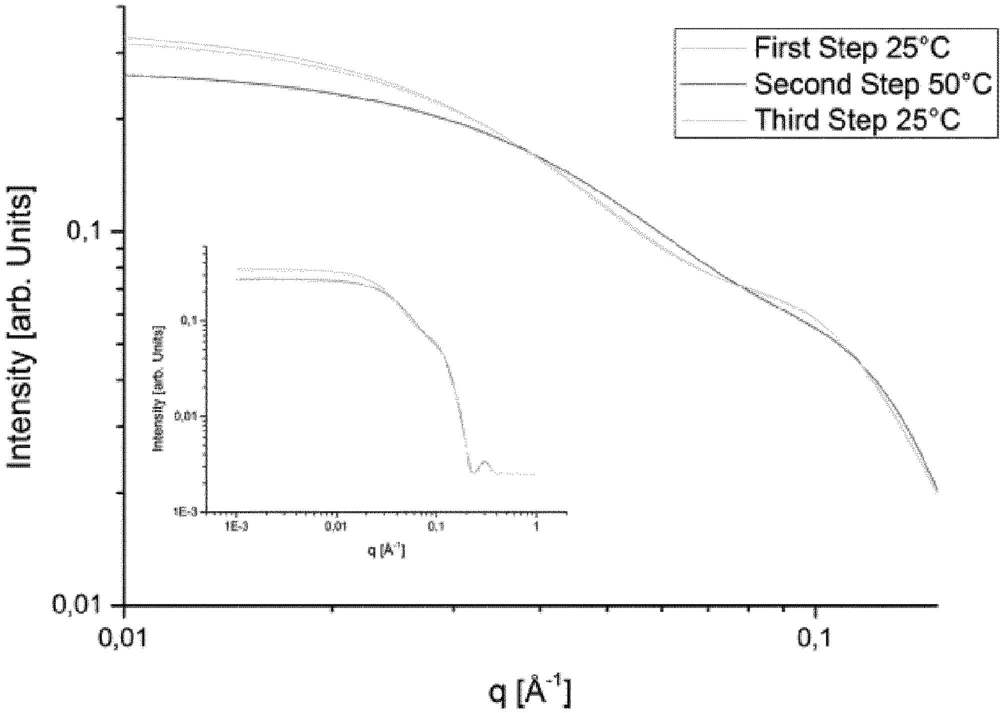
First Step 25°C
Second Step 50°C
Third Step 25°C
Intensity [arb. Units]
0,1
0,01
0,01
0,1
q [Å-1]
Intensity [arb. Units]
0,1
0,01
1E-3
1E-3
0,01
0,1
1
q [Å-1]

Fig. 16

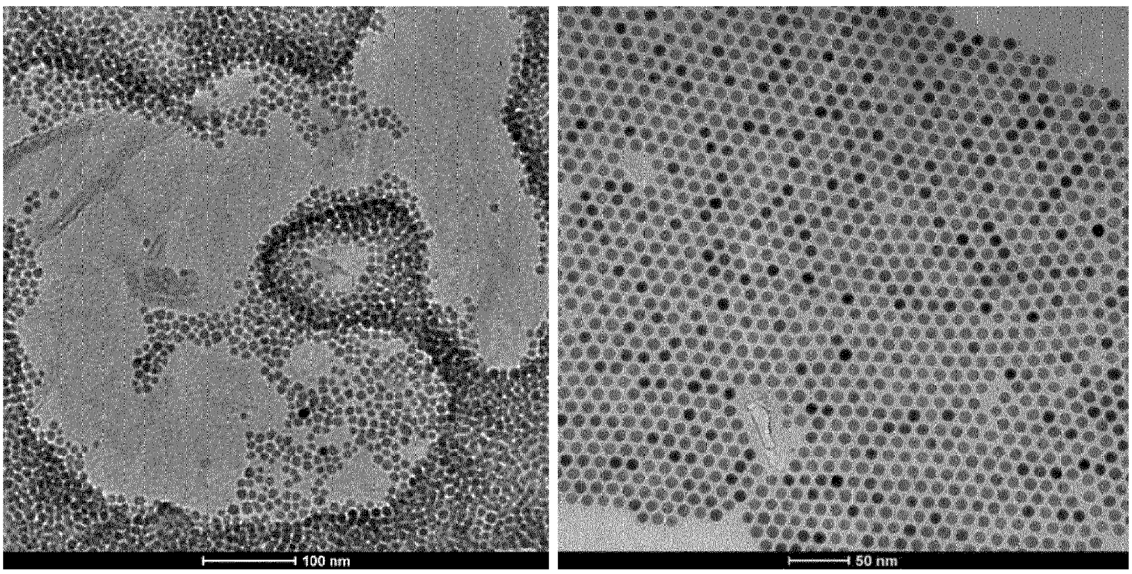
100 nm
50 nm

Europäisches Patentamt
European Patent Office
Office européen des brevets

# EUROPEAN SEARCH REPORT

Application Number
EP 14 18 0393

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2013/164222 A1 (HYEON TAEG HWAN [KR] ET AL) 27 June 2013 (2013-06-27)<br>* claims 1-16 *<br>* paragraphs [0035] - [0037] *<br>* examples 1-10, 12, 13, 17-20 *<br>----- | 1,3-15<br>2 | INV.<br>C01G49/06<br>A61K49/18<br>C01G49/08<br>C09C3/10<br>C09C1/24 |
| X<br>A | WO 2013/110828 A1 (SOLUCIONES NANOTECNOLOGICAS S L [ES]; CERDAN GARCIA-ESTELLER SEBASTIAN) 1 August 2013 (2013-08-01)<br>* claims 1-10 *<br>* paragraphs [0017] - [0027], [0030] *<br>----- | 1,3-15<br>2 | |
| X<br>A | EP 0 516 252 A2 (DIAGNOSTIKFORSCHUNG INST [DE]) 2 December 1992 (1992-12-02)<br>* claims 1-11 *<br>* examples 2,4-14 *<br>----- | 1,3-15<br>2 | |
| A | ESTHER AMSTAD ET AL: "Ultrastable Iron Oxide Nanoparticle Colloidal Suspensions Using Dispersants with Catechol-Derived Anchor Groups",<br>NANO LETTERS,<br>vol. 9, no. 12,<br>9 December 2009 (2009-12-09), pages 4042-4048, XP55023825,<br>ISSN: 1530-6984, DOI: 10.1021/nl902212q<br>* the whole document *<br>-----<br>-/-- | 1-15 | TECHNICAL FIELDS SEARCHED (IPC)<br>C01G<br>A61K<br>C09C |

The present search report has been drawn up for all claims

1

| Place of search | Date of completion of the search | Examiner |
|---|---|---|
| The Hague | 20 January 2015 | Gerwann, Jochen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 0393

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JEFFREY L DALSIN ET AL: "Protein resistance of titanium oxide surfaces modified by biologically inspired mPEG-DOPA", LANGMUIR, AMERICAN CHEMICAL SOCIETY, NEW YORK, NY; US , vol. 21, no. 2 1 February 2005 (2005-02-01), pages 640-646, XP003030998, ISSN: 0743-7463, DOI: 10.1021/LA048626G Retrieved from the Internet: URL:http://pubs.acs.org/doi/abs/10.1021/la048626g [retrieved on 2004-12-09] * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 20 January 2015 | Gerwann, Jochen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 18 0393

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2015

| Patent document cited in search report | Publication date | Patent family member(s) | Publication date |
|---|---|---|---|
| US 2013164222 A1 | 27-06-2013 | AU 2011286527 A1 | 22-11-2012 |
| | | CA 2797101 A1 | 09-02-2012 |
| | | CN 103153348 A | 12-06-2013 |
| | | EP 2600903 A2 | 12-06-2013 |
| | | JP 2013534893 A | 09-09-2013 |
| | | RU 2013109406 A | 10-09-2014 |
| | | US 2013164222 A1 | 27-06-2013 |
| | | WO 2012018240 A2 | 09-02-2012 |
| WO 2013110828 A1 | 01-08-2013 | EP 2808036 A1 | 03-12-2014 |
| | | WO 2013110828 A1 | 01-08-2013 |
| EP 0516252 A2 | 02-12-1992 | AT 156021 T | 15-08-1997 |
| | | AU 653220 B2 | 22-09-1994 |
| | | CA 2068632 A1 | 29-11-1992 |
| | | DE 4117782 A1 | 03-12-1992 |
| | | DE 59208743 D1 | 04-09-1997 |
| | | DK 0516252 T3 | 25-08-1997 |
| | | EP 0516252 A2 | 02-12-1992 |
| | | ES 2106134 T3 | 01-11-1997 |
| | | GR 3024898 T3 | 30-01-1998 |
| | | IE 921595 A1 | 02-12-1992 |
| | | IL 101929 A | 26-08-1994 |
| | | JP 3306810 B2 | 24-07-2002 |
| | | JP H07122410 A | 12-05-1995 |
| | | NO 921767 A | 30-11-1992 |
| | | NZ 242669 A | 28-08-1995 |
| | | US 5427767 A | 27-06-1995 |
| | | ZA 9203299 A | 30-12-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011026572 A1


### Non-patent literature cited in the description


- **J. L. DALSIN et al.** *Messersmith, Langmuir,* 2004, vol. 21, 640-646
- **PARK et al.** *Nat. Mater.,* 2004, vol. 3, 891-895
- **E. AMSTAD et al.** *Nano Letters,* 2009, vol. 9, 4042-4048
- **E. AMSTAD et al.** *J. Phys. Chem. C,* 2011, vol. 115, 683-691
- **T. HYEON et al.** *Journal of the American Chemical Society,* 2001, vol. 123, 12798-12801
- **S.-J. PARK et al.** *J. Am. Chem. Soc.,* 2000, vol. 122, 8581-8582
- **E. KANG et al.** *Journal of Physical Chemistry B,* 2004, vol. 108, 13932-13935
- **J. PARK et al.** *Nat. Mater.,* 2004, vol. 3, 891-895
- **J. PARK et al.** *Angew Chem Int Ed Engl,* 2005, vol. 44, 2873-2877
- **S. G. KWON et al.** *J. Am. Chem. Soc.,* 2007, vol. 129, 12571-12584
- **J. PARK et al.** *Angew. Chem., Int. Ed.,* 2007, vol. 46, 4630-4660
- **C. CAVELIUS et al.** *Crystal Growth and Design,* 2012, vol. 12, 5948-5955